(19) Europäisches Patentamt
European Patent Office
Office européen des brevets

(11) **EP 3 724 329 B1**

(12) **EUROPEAN PATENT SPECIFICATION**

(45) Date of publication and mention
of the grant of the patent:
**20.08.2025 Bulletin 2025/34**

(21) Application number: **18815208.6**

(22) Date of filing: **12.12.2018**

(51) International Patent Classification (IPC):
*C12N 9/48* (2006.01)     *C12N 11/00* (2006.01)
*A23J 3/34* (2006.01)     *C12P 21/06* (2006.01)

(52) Cooperative Patent Classification (CPC):
**C12N 9/485; A23J 3/34; C12N 11/08; C12N 11/14;**
**C12P 21/06; C12Y 304/11; C12Y 304/11001**

(86) International application number:
**PCT/EP2018/084525**

(87) International publication number:
**WO 2019/115603 (20.06.2019 Gazette 2019/25)**

(54) **AMINOPEPTIDASE AND ITS USES**

**AMINOPEPTIDASE UND DEREN VERWENDUNGEN**

**AMINOPEPTIDASE ET SES UTILISATIONS**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **12.12.2017 EP 17306756**

(43) Date of publication of application:
**21.10.2020 Bulletin 2020/43**

(73) Proprietors:
• **Centre National de la Recherche Scientifique**
**75016 Paris (FR)**
• **Commissariat à l'Energie Atomique et aux**
**Energies**
**Alternatives**
**75015 Paris (FR)**
• **Université Grenoble Alpes**
**38400 Saint-Martin-d'Hères (FR)**

(72) Inventors:
• **FRANZETTI, Bruno**
**38360 Sassenage (FR)**
• **GIRARD, Eric**
**26100 Romans-Sur-Isère (FR)**
• **APPOLAIRE, Alexandre**
**38000 Grenoble (FR)**
• **BASBOUS, Hind**
**38000 Grenoble (FR)**

(74) Representative: **Novagraaf Technologies**
**Bâtiment O2**
**2, rue Sarah Bernhardt**
**CS90017**
**92665 Asnières-sur-Seine Cedex (FR)**

(56) References cited:
• **DATABASE UniProt [online] 1 November 1997**
**(1997-11-01), "RecName: Full=Putative**
**aminopeptidase MJ0555; EC=3.4.11.-;",**
**XP002780483, retrieved from EBI accession no.**
**UNIPROT:Q57975 Database accession no.**
**Q57975**
• **ALEXANDRE APPOLAIRE ET AL: "TET**
**peptidases: A family of tetrahedral complexes**
**conserved in prokaryotes", BIOCHIMIE, vol. 122,**
**4 November 2015 (2015-11-04), pages 188 - 196,**
**XP029410761, ISSN: 1638-6183, DOI: 10.1016/**
**j.biochi.2015.11.001**

Remarks:
The complete document including Reference
Table(s) and the Sequence Listing(s) can be
downloaded from the EPO website

**Description**

The present invention relates to the use of a peptidase and its use. Aminopeptidases represent large class of enzymes that hydrolyze peptide bonds between amino acids in protein or peptide chain. They are involved in broad array of cellular functions, from protein destruction to post-translational modifications of proteins and peptides.

During the last decade, it has been found that several enzymes from M42 and M18 metallopeptidase families self-assembled in half megadalton complexes made of twelve subunits. These proteins were named TET because of the tetrahedral shape adopted by these dodecameric edifices.

TET complex was first purified from halophilic archaea during a search for protein quality control machines involved in low salt stress. This novel type of cage-forming protease attracted interest since it is present in many cell types and has homologues in all kingdoms of life. Previous structural studies revealed that all TET dodecamers share similar architectures: the twelve monomers are associated in a tetrahedral particle with four large substrate entry pores formed by the junction of six subunits situated on each facet and four catalytic chambers each comprising three active sites of three monomers which formed the apex of tetrahedron. This architecture is strikingly different from the barrel-shaped one adopted by other cytosolic compartmentalized peptidases. TET assembling mechanism from dimeric precursors was determined using a combination of biophysical techniques. They demonstrated that it is a highly ordered process involving predominantly hexameric precursors.

Biochemical characterizations of TET complex in vivo and in vitro indicated that the purpose of self-compartmentalization in the M42 peptidases is to trigger their amidolytic activity toward long peptides. Different types of TET enzymes were found in archaea, bacteria and eukarya and different versions of enzymatic complexes often co-exist in the cytosol of the same organism.

All M42 TET complexes characterized so far are cobalt-activated aminopeptidases able to degrade oligopeptides to single amino acids (Appolaire et al. (2015) Biochimie 122:188-196). However, enzymatic studies revealed that they displayed marked substrate preferences. This aspect of TET biochemistry was mostly studied in Pyrococcus horikoshii, a deep-sea hyperthermophilic Euryarchaeota that contains three different versions of TET complex: PhTET1, a glutamyl-aspartyl aminopeptidase, PhTET2, a leucyl aminopeptidase with broad activity against neutral amino acids, and PhTET3, a lysyl aminopeptidase.

In these complexes, the comparison of the "S1" binding pocket properties supports a clear correlation between charge distribution and substrate specificities.

Despite important progress in determining the structure-function relationship of TET machinery, many fundamental questions remain about the cellular function and the mode of action of TET system. In particular, it is not fully understood the detailed mechanism of polypeptide substrate filtering and guidance to the active site as well as the amino acid-product expulsion system remains elusive.

A genomic survey on the occurrence of TET-like enzymes in prokaryotic genomes revealed that some archaea possess up to four versions of TET proteins while others only express one version. In order to specify the basic physiological roles of TET system and the reasons underlying the multiplicity of TET cellular machines in some microorganisms and to bring further insights into their structure-function relationship, the inventors studied the biochemical and structural properties of the unique TET complex from Methanocaldococcus jannaschii, a deep-sea hyperthermophilic archaea. So there is a need to provide new peptidases for industry.

The invention relates to the use of a TET protein as a N-terminus aromatic amino acid residues specific exopeptidase, said TET protein consisting of the amino acid sequence as set forth in SEQ ID NO: 1, or any homologous protein derived from said TET protein as set forth in SEQ ID NO: 1 by substitution, addition or deletion of at least one amino acid, provided that the derived protein retains at least 70 % of identity with the amino acid sequence as set forth in SEQ ID NO: 1, and said derived protein retaining a N-terminus aromatic amino acid residues specific exopeptidase activity.

The invention is based on the characterization by the inventors of the enzymatic activity of MjTET. This characterization revealed that this archaeal TET aminopeptidase displayed broad substrate specificity, thus indicating a housekeeping function devoted to the non-specific destruction of polypeptides. More advantageously, the inventors identified that MjTET is a TET protein having a specificity toward the N-terminus aromatic amino acids, said activity vis-à-vis aromatic residues being largely enhanced compared to the other TET protein that where characterized to date.

Moreover, the inventors also identify that the protein according to the invention harbors an aminopeptidase activity toward all the amino acids except aspartic acid, cysteine and proline. This means that if these amino acids are present in the N-terminus of a peptide, a polypeptide or a protein, the protein according to the invention will not be able to brake the peptidic bound.

Advantageously, in another aspect, the invention relates to the use of a TET protein as a N-terminus exopeptidase provided that the amino acid at the N-terminus is not aspartic acid, cystein or proline, said TET protein consisting of the amino acid sequence as set forth in SEQ ID NO: 1, or any homologous protein derived from said TET protein as set forth in SEQ ID NO: 1 by substitution, addition or deletion of at least one amino acid, provided that the derived protein retains at least 70 % of identity with the amino acid sequence as set forth in SEQ ID NO: 1, and said derived protein retaining a N-terminus specific exopeptidase activity.

The inventors were also able to identify the structure of the protein. Structural analysis combining X-ray crystallography and cryo-electron microscopy allowed providing complete MjTET atomic model. In this model, internal network of mobile loops that were poorly defined in previous crystallographic studies could be observed. The topological analysis of these loops highlights an elegant peptide guiding system toward catalytic sites.

In the invention the protein comprising the amino acid sequence SEQ ID NO : 1 is called MjTET.

The invention also relates to the use of at least a TET protein harboring at least a N-terminus aromatic amino acid residues specific exopeptidase activity, for the modification of all or part of the polypeptide content of a substrate comprising peptides, polypeptides and/or proteins, said at least a TET protein consisting of the amino acid sequence as set forth in SEQ ID NO: 1,

or any homologous protein derived from said at least a TET protein as set forth in SEQ ID NO: 1 by substitution, addition or deletion of at least one amino acid, provided that the derived protein retains at least 70 % of identity with the amino acid sequence as set forth in SEQ ID NO: 1, and said derived protein retaining a N-terminus aromatic amino acid residues specific exopeptidase activity.

By "exopeptidase", it is meant in the invention a peptidase that catalyses the cleavage of the terminal peptide bond of an amino acid chain, starting either from the amino or carboxyl terminal of the said amino acid chain.

By "peptide", it is meant in the invention an amino acid chain comprising at least 2 amino acids. Peptides can be obtained either from protein degradation or from chemical synthesis. By "polypeptide", it is meant in the invention an amino acid chain larger than a peptide and obtained from degradation of proteins and not from chemical synthesis. Peptides and polypeptides may harbour biological function, but said function is not associated with a cellular process. By "protein", it is meant in the invention an amino acid chain containing molecule harbouring biological function and which is found naturally in an organism, said biological function being part of a natural process of the cell.

By "at least 70% of identity with the sequence as set forth in SEQ ID NO : 1", it is meant in the invention 70 %, 71 %, 72 %, 73 %, 74 %, 75 %, 76 %, 77 %, 78 %, 79 %, 80 %, 81 %, 82 %, 83 %, 84 %, 85 %, 86 %, 87 %, 88 %, 89 %, 90 %, 91 %, 92 %, 93 %, 94 %, 95 %, 96 %, 97 %, 98 %, 99 % and 100 % of identity with the sequence as set forth in SEQ ID NO : 1. Regarding the percentage of identity, it is defined by the percentage of amino acid residues of SEQ ID NO : 1 which align with the same amino acid in the sequence of the homologous protein. The sequence alignment is carried out using dedicated algorithms and programs (such as ClustalW, for instance).

By "modification of all or part of the polypeptide", it is meant in the invention that the modification of a peptide can result in the removal of one or more amino acid from the peptide. If the peptide contains only aromatic amino acids, the peptide can be completely degraded, i.e. can be converted into the free amino acids that constitute the peptide.

Moreover, the "modification of all or part of the polypeptide", means also in the invention that, if a composition contains two or more peptides, at least one peptide will be degraded by contacted the TET protein according to the invention. If only some peptides are degraded, the composition of peptide will be considered to be partially modified. If all the peptides are subjected to a degradation, the composition of peptide will be considered to be totally modified.

In the invention, regarding a peptide, a polypeptide, a protein or a polypeptide content, the terms "modification" and "degradation" can be used uniformly. In the invention, the term "comprising" is meant to include the terms "consisting essentially of" and "consisting of".

The aromatic amino acids encompassed by the scope of the invention are phenylalanine (Phe; F), tryptophan (Trp; W), tyrosine (Tyr; Y) and histidine (His; H). Thus according to the invention, if a peptide, a polypeptide or a protein contain in its amino-terminal part one amino acid chosen among F, W, Y and H, said amino acid could be released from the peptide by contacting the TET protein, according to the invention, i.e. the MjTET protein.

Advantageously, the invention relates to the use as defined above, wherein said TET protein or said derived protein originates from an extremophile microorganism belonging to the *Methanococcales* order and are isolated from this extremophile microorganism.

By "extremophile", it is meant in the invention an organism that thrives in physically or geochemically extreme conditions that are detrimental to most life on Earth. In contrast, organisms that live in more moderate environments may be termed mesophiles or neutrophiles. The order Methanococcales consists of 4 genera, Methanocaldococcus (hyperthermophiles), Methanotorris (extreme thermophiles), Methanothermococcus (moderate thermophiles) and Methanococcus (mesophiles).

The Methanococcales are strictly oxygen-intolerant and obtain energy by reducing $CO_2$ with $H_2$ to generate methane. They can also scavenge small organic molecules. They move with two bundles of flagella.

The first species of *Methanococcales* to be isolated came from a sediment sample collected from the sea floor. The sample was found at the base of a 8600-foot deep "white smoker" chimney located on the east Pacific Rise.

Advantageously, the invention relates to the use as defined above, wherein said extremophile microorganism is *Methanocaldocuccus jannaschii*.

*Methanococcus jannaschii* is an autotropic hyperthermophillic organism that belongs to the kingdom of Archaea.

They were found to live in extreme environments such as hydrothermal vents at the bottom of the oceans in which water reaches boiling temperature or pressure is extremely high.

Advantageously, the invention relates to the use as defined above, wherein peptides, polypeptides and/or proteins of said substrate are obtained from food, chemical and health industries, or from natural biomass.

The generation of protein-rich industrial wastes is very high (only from sunflower, about one million tons in Spain). These wastes are not used at all, or are underused in the form of low added-value products. This type of by-products constitutes a reservoir of proteins with a great economic potential.

The TET protein according to the invention, in view of its activity, can be used in various domains for instance, but without limitation:

- For the valorization of agriculture wastes: proteins and peptides originating from agriculture can be recycled for animal feed, or for producing feed additives. In order to be used, the vegetal proteins have to be degraded to avoid any antinutritional side effects.
- For the valorization of chemical wastes
- For the valorization of food industry waste: suitable protein to be treated with the protein according to the invention, i.e. MjTET, may be for instance proteins obtained from dairy products, fruit juices, beers, flours or cured products. Moreover, the products from wine industry, from cheese industry, and see food industry are particularly advantageous and can be valorized by using the TET protein according to the invention.
- Products from biomass: some alternative to common protein sources are now emerging in view of the need to provide more and more feed for the Earth population. For instance, algae and microorganisms, along with insects, are very rich in protein that can be used for providing new sources of amino acids or proteins that can be eaten by animal and humans. Therefore, proteins or peptides from algae or microorganisms can be relevant sources for treatment by using the TET protein according to the invention.
- Another kind of wastes are the wastes produced from health industry. For instance, solid, regulated medical waste can includes materials generated in the diagnosis, treatment, research, or immunization of human beings or animals. Examples of regulated medical waste includes: cultures and stocks, pathological wastes, human blood and blood products, sharps, certain animal waste and isolation wastes. The TET protein according to the invention may help to valorize such kinds of wastes.

The invention also relates to a method for degrading, from the N-terminus part, a polypeptide harboring an aromatic residue at its N-terminal part, said method comprising a step of contacting said polypeptide harboring an aromatic residue at its N-terminal part with

- at least a TET protein harboring at least a N-terminus aromatic amino acid residues specific exopeptidase activity, said TET protein consisting of the amino acid sequence as set forth in SEQ ID NO: 1,
- or any homologous protein derived from said TET protein as set forth in SEQ ID NO: 1 by substitution, addition or deletion of at least one amino acid, provided that the derived protein retains at least 70 % of identity with the amino acid sequence as set forth in SEQ ID NO: 1, and said derived protein retaining a N-terminus aromatic amino acid residues specific exopeptidase activity,

and possibly a step of recovering the resulting N-terminal aromatic amino acid residue free peptides.

According to the invention, in order to carry out a degradation of a peptide, a polypeptide or a protein, which contains aromatic amino acids in its N-terminus part, the TET protein or the homologous protein of said TET protein is contacted with the peptide to be degraded. The TET protein and the substrate are incubated in an appropriated buffer, as disclosed in the Example.

It is advantageous, when the resulting peptide, polypeptide or protein has been degraded, resulting from the action of the TET protein, to recover, or purify said resulting N-terminal aromatic amino acid residue free peptide, polypeptide or protein. From his general knowledge, the skilled person can easily carry out such purification step, for instance by using chromatography methods or immunological methods.

The invention also relates to a method for modifying all or part of the polypeptide content of a substrate comprising peptides, polypeptides and/or proteins, said method comprising a step of contacting said substrate with

- at least a TET protein harboring at least a N-terminus aromatic amino acid residues specific exopeptidase activity, said TET protein consisting of the amino acid sequence as set forth in SEQ ID NO: 1,
- or any homologous protein derived from said TET protein as set forth in SEQ ID NO: 1 by substitution, addition or deletion of at least one amino acid, provided that the derived protein retains at least 70 % of identity with the amino acid sequence as set forth in SEQ ID NO: 1, and said derived protein retaining a N-terminus aromatic amino acid residues specific exopeptidase activity,

and possibly a step of recovering the modified polypeptide content of a substrate.

Advantageously, the invention relates to the method, or the use, as defined above, wherein said step of contacting is carried out at a pH range varying from 6.5 to 10.

As demonstrated in the examples, the inventors identified that the TET protein according to the invention is stable in a large range of pH and is active on its substrate from acid medium to basic medium. The TET protein according to the invention is active at pH 6.5, 7, 7.5, 8, 8.5, 9, 9.5 or 10. The optimal pH range is from 7,5 to 9.5, wherein the activity of the protein varies from 80% to 100% of the optimal activity, measured as the $\mu$mol of substrat per mg of MjTET per min.

Advantageously, the invention relates to the method, or the use as defined above, wherein said step of contacting is carried out at a temperature range varying from 80°C to 100°C.

As a thermostable protein, the TET protein according to the invention is active, i.e. can catalyse the degradation of a peptide, a polypeptide or a protein, by removing amino terminus aromatic amino acids residues at high temperatures varying from 80°C to 100°C. This means that the protein is active at the following temperatures 80 °C, 80.1 °C, 80.2 °C, 80.3 °C, 80.4 °C, 80.5 °C, 80.6 °C, 80.7 °C, 80.8 °C, 80.9 °C, 81 °C, 81.1 °C, 81.2 °C, 81.3 °C, 81.4 °C, 81.5 °C, 81.6 °C, 81.7 °C, 81.8 °C, 81.9 °C, 82 °C, 82.1 °C, 82.2 °C, 82.3 °C, 82.4 °C, 82.5 °C, 82.6 °C, 82.7 °C, 82.8 °C, 82.9 °C, 83 °C, 83.1 °C, 83.2 °C, 83.3 °C, 83.4 °C, 83.5 °C, 83.6 °C, 83.7 °C, 83.8 °C, 83.9 °C, 84 °C, 84.1 °C, 84.2 °C, 84.3 °C, 84.4 °C, 84.5 °C, 84.6 °C, 84.7 °C, 84.8 °C, 84.9 °C, 85 °C, 85.1 °C, 85.2 °C, 85.3 °C, 85.4 °C, 85.5 °C, 85.6 °C, 85.7 °C, 85.8 °C, 85.9 °C, 86 °C, 86.1 °C, 86.2 °C, 86.3 °C, 86.4 °C, 86.5 °C, 86.6 °C, 86.7 °C, 86.8 °C, 86.9 °C, 87 °C, 87.1 °C, 87.2 °C, 87.3 °C, 87.4 °C, 87.5 °C, 87.6 °C, 87.7 °C, 87.8 °C, 87.9 °C, 88 °C, 88.1 °C, 88.2 °C, 88.3 °C, 88.4 °C, 88.5 °C, 88.6 °C, 88.7 °C, 88.8 °C, 88.9 °C, 89 °C, 89.1 °C, 89.2 °C, 89.3 °C, 89.4 °C, 89.5 °C, 89.6 °C, 89.7 °C, 89.8 °C, 89.9 °C , 90 °C, 90.1 °C, 90.2 °C, 90.3 °C, 90.4 °C, 90.5 °C, 90.6 °C, 90.7 °C, 90.8 °C, 90.9 °C, 91 °C, 91.1 °C, 91.2 °C, 91.3 °C, 91.4 °C, 91.5 °C, 91.6 °C, 61.7 °C, 91.8 °C, 91.9 °C, 92 °C, 92.1 °C, 92.2 °C, 92.3 °C, 92.4 °C, 92.5 °C, 92.6 °C, 92.7 °C, 92.8 °C, 92.9 °C, 93 °C, 93.1 °C, 93.2 °C, 93.3 °C, 93.4 °C, 93.5 °C, 93.6 °C, 93.7 °C, 93.8 °C, 93.9 °C, 94 °C, 94.1 °C, 94.2 °C, 94.3 °C, 94.4 °C, 94.5 °C, 94.6 °C, 94.7 °C, 94.8 °C, 94.9 °C, 95 °C, 95.1 °C, 95.2 °C, 95.3 °C, 95.4 °C, 95.5 °C, 95.6 °C, 95.7 °C, 95.8 °C, 95.9 °C, 96 °C, 96.1 °C, 96.2 °C, 96.3 °C, 96.4 °C, 96.5 °C, 96.6 °C, 96.7 °C, 96.8 °C, 96.9 °C, 97 °C, 97.1 °C, 97.2 °C, 97.3 °C, 97.4 °C, 97.5 °C, 97.6 °C, 97.7 °C, 97.8 °C, 97.9 °C, 98 °C, 98.1 °C, 98.2 °C, 98.3 °C, 98.4 °C, 98.5 °C, 98.6 °C, 98.7 °C, 98.8 °C, 98.9 °C, 99 °C, 99.1 °C, 99.2 °C, 99.3 °C, 99.4 °C, 99.5 °C, 99.6 °C, 99.7 °C, 99.8 °C, 99.9 °C and 100°C.

These results are shown in the Example.

Advantageously, the invention relates to the method, or the use as defined above, wherein said step of contacting is carried out in presence of cobalt ions.

The inventors also shown that the TET protein according to the invention is more active when Cobalt ions are present as cofactor that activate the enzyme.

Thus, and more advantageously, the invention relates to the method, or the use, as defined above, wherein the step of contacting is carried out :

- in presence of cobalt ions and at a temperature varying from 80°C to 100°C, or
- in presence of cobalt ions at a pH range varying from 6.5 to 10, or
- at a temperature range varying from 80°C to 100°C and a pH range varying from 6.5 to 10, or
- in presence of cobalt ions and at a temperature range varying from 80°C to 100°C and a pH range varying from 6.5 to 10.

Compared to other TET family member peptidases, the TET protein according to the invention is active at a pressure varying from 0,1 MPa to 350 MPa.

Advantageously, the invention relates to the above method, wherein said at least a TET protein or said derived protein is immobilized on a solid support, preferably on a filter cartridge, silica, or magnetic beads.

In view of the robustness of the TET protein, and the stability of the structure, it is possible to immobilized the TET protein on a support. Such a support is advantageous and allows to carry out a peptide, polypeptide or peptide degradation and recover the resulting degraded peptide, polypeptides and protein, easily without additional step of separation of the enzyme and the resulting product.

In another advantageous embodiment, the invention relates to the method, or the use as defined above, wherein said at least a TET protein or said derived protein is expressed in a mesophilic host, from a plasmid, and then purified from cell lysates by a thermal denaturation step of the host proteins.

In order to produce the TET protein, and carry the method above defined, it is possible to produce the TET protein in a mesophilic host, for instance by overexpression of the TET protein the nucleic acid sequence of which being contained in a plasmid, or a vector. By culturing the mesophilic host defined above, it is possible to recover, i.e. purify, the TET protein according to the invention by lysis protocols well known in the art. The TET protein can also be easily purified by denaturing the proteins of the host at a temperature where the TET protein remains active, from 60°C to 90°C.

The invention also relates to the use of a solid support for the modification of all or part of the polypeptide content of a substrate comprising peptides, polypeptides and/or proteins, wherein is immobilized on said solid support:

- at least a TET protein harboring at least a N-terminus aromatic amino acid residues specific exopeptidase activity, said TET protein consisting of the amino acid sequence as set forth in SEQ ID NO: 1,
- or any homologous protein derived from said TET protein as set forth in SEQ ID NO: 1 by substitution, addition or deletion of at least one amino acid, provided that the derived protein retains at least 70 % of identity with the amino acid sequence as set forth in SEQ ID NO: 1, and said derived protein retaining a N-terminus aromatic amino acid residues specific exopeptidase activity.

Advantageously, the invention relates to the use as defined above, wherein said solid support is a filter cartridge, silica or magnetic beads.

Advantageously, said solid support is a filter cartridge or silica beads or magnetic beads or on organic polymeric materials, or on inorganic polymeric materials or on membrane devices or in microcapsules. Membrane devices include hollow fibres.

Alternatively, said at least TET protein or said derived protein is immobilized as crosslinked enzyme aggregates (CLEAs).

CLEAs are developed by precipitation of the enzyme from a solution by adding salt, such as ammonium sulphate, or water-miscible organic solvent, followed by crosslinking with a bifunctional reagent (Bilal M, Iqbal HM, Guo S, Hu H, Wang W, Zhang X (2017) State-of-the-art protein engineering approaches using biological macromolecules: A review from immobilization to implementation view point. Int J Biol Macromol. Nov 2.).

Advantageously, the invention relates to the use as defined above, wherein peptides, polypeptides and/or proteins of said substrate are obtained from food, chemical and health industries, or from biomass, as above mentioned. Advantageously, peptides, polypeptides and/or proteins of said substrate are obtained from food, chemical and health industries, or from biomass, as mentioned above. Advantageously, proteins from fermented products or soya products or sea food products or cheese products.

The invention will be better understood from the following examples and the 12 following figures

## Legends to the figures

**Figure 1** is a photography by negative stain electron microscopy at 49000X magnification of MjTET. The enzyme displays hollow tetrahedral-shaped particles.

**Figures 2A-C** are graphs representing the influence of pH, metal ion and temperature variations on MjTET specific activities.

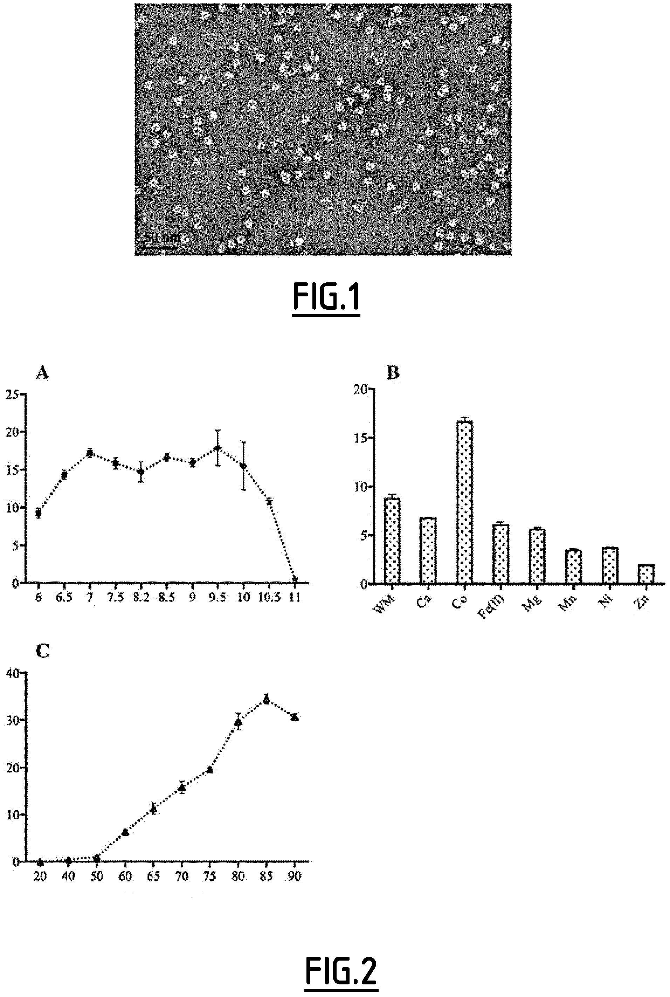

Figure 2A is a graph showing the evolution of the MjTET specific activity ($\mu$mol(pNA) mg$^{-1}$ (MjTET) min$^{-1}$) as function of pH in PIPES (■), CHES (♦) and CAPS ( ) buffers.

Figure 2B is a graph showing the modifications of MjTET specific activity ($\mu$mol(pNA) mg$^{-1}$ (MjTET) min$^{-1}$) in absence (WM: without metal) or in presence of different metal ions as indicated.

Figure 2C. is a graph showing the effect of the temperature increasing on MjTET leucyl aminopeptidase activity ($\mu$mol(pNA) mg$^{-1}$ (MjTET) min$^{-1}$).

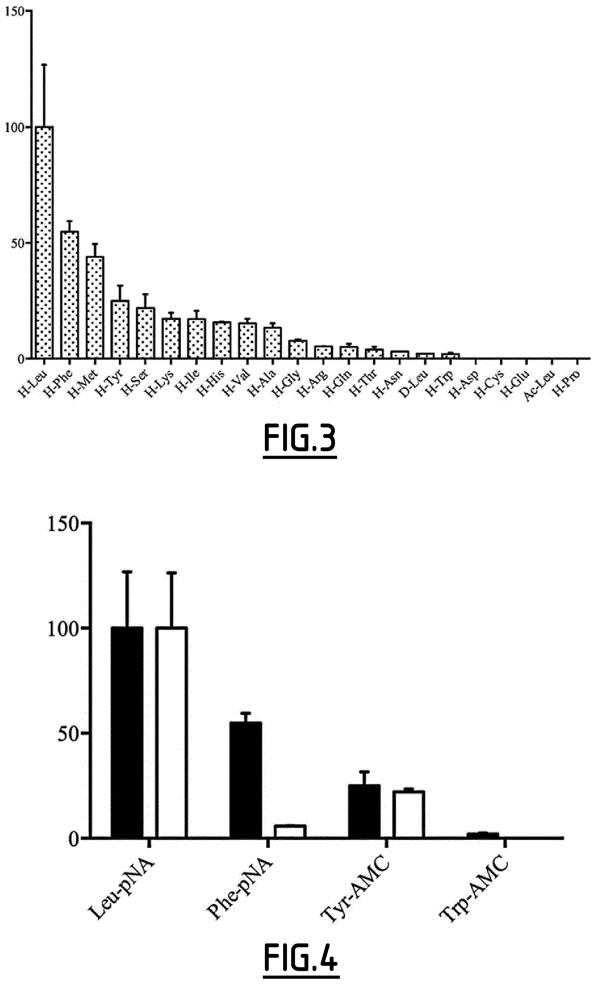

**Figure 3** is a graph representing the relative activity (%) of MjTET on substrate. MjTET displays broad substrate preference and higher activity was measured against hydrophobic residues: leucine, phenylalanine and methionine.

**Figure 4** is a graph showing the comparison of MjTET and PhTET2 amidolytic activities against aromatic residues. MjTET (black colums) shows higher capacity to hydrolyze phenylalanine and tyrosine in comparison to PhTET2 (white columns). However these enzymes are unable to cleave tryptophan from substrate Nt end.

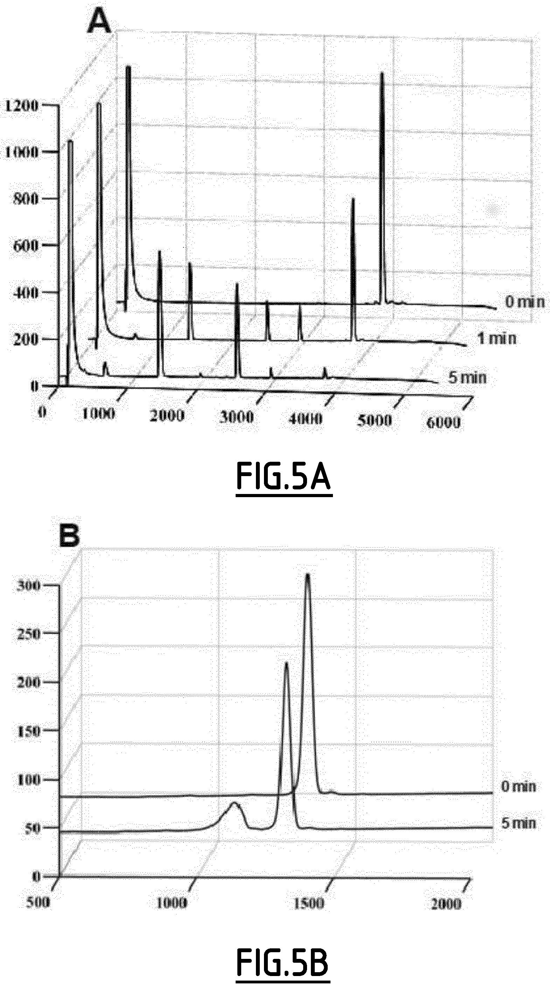

**Figures 5A-B** represents chromatographic profiles of peptide degradations by MjTET, obtained from reverse phase HPLC.

Figure 5A is a chromatographic representation of H-Trp-Phe-Tyr-Ser(PO3H2)-Pro-Arg-pNA hydrolysis by MjTET after 1 min and 5 min of incubation.

Figure 5B is a chromatographic profile of H-Asp-Ala-Tyr-Pro-Ser degradation by MjTET after 5 min of incubation.

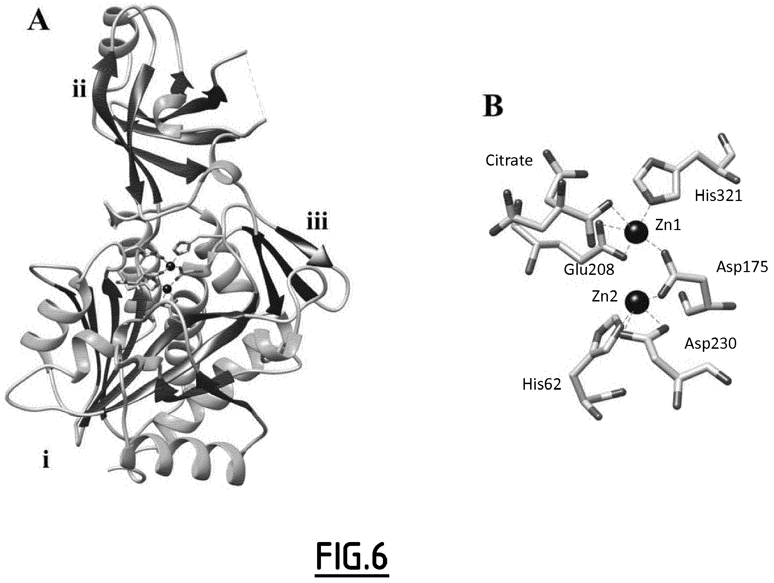

**Figures 6A-B** are representations of the X-ray structure of MjTET monomer.

Figure 6A is a representation of the tertiary structure of MjTET monomer. i. Catalytic domain formed by the bigger $\alpha/\beta$ globular domain. ii. Smaller $\alpha/\beta$ globular that formed dimerization domain (I). Residues 115-124 carried by the dimerization domain I, are absent. iii. Three-stranded antiparallel $\beta$-sheet that formed the dimerization domain II.

Figure 5B is a close-up view of MjTET bimetallic active site. Two zinc ions (black spheres) are modeled in the active. Zn1 is coordinated by Asp175, Glu208, His321 and citrate molecule, while His62, Asp175 and Asp230 coordinate Zn2.

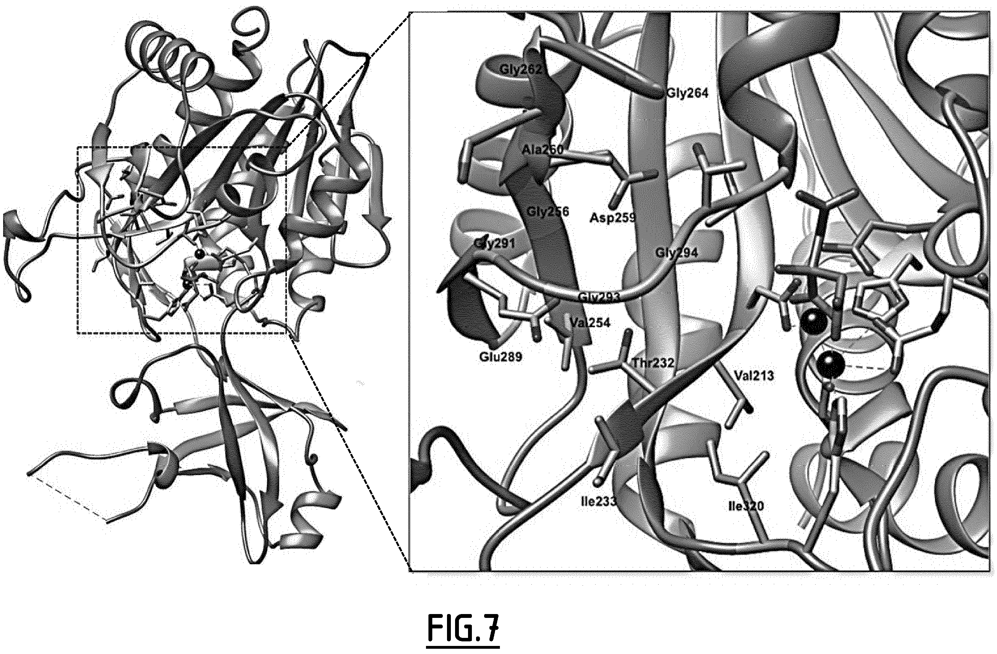

**Figure 7** is a representation of a view of MjTET specificity pocket near the active site. Hydrophobic and neutral residues (represented by purple sticks) define MjTET specificity pocket allowing the reception of side chains of hydrophobic and neutral residues. Furthermore, two negative amino acids (Asp259, Glu289) are also present in this pocket, thus explaining the capacity of MjTET to hydrolyze positive substrates.

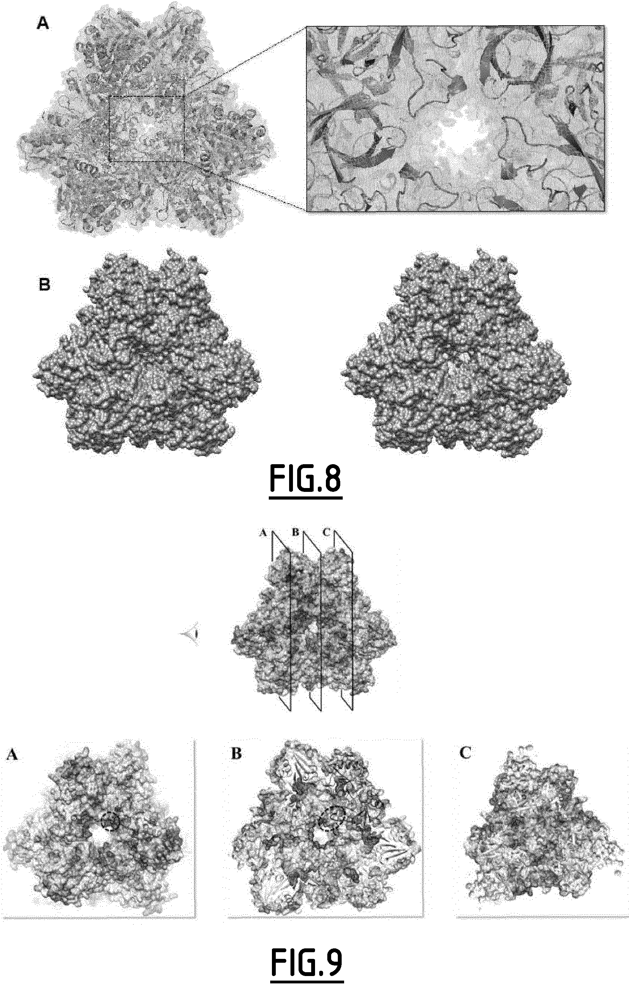

**Figures 8A-B** show a representation of the X-ray missing loops in the cryo-EM map and inside the surface of MjTET particle.

Figure 8A is a view of the internal electron density obtained by cryo-EM, where the X-ray atomic model of MjTET (blue) is fitted inside and the X-ray missing loops (yellow) were built.

Figure 8B is a surface representation of X-ray structure of MjTET without (left) and with (right) internal loops (yellow) built from cryo-EM map. Clear obstruction of the internal cavity is observed for MjTET model enclosing these mobile loops.

**Figures 9A-C** represent MjTET electrostatic potential observed at three different levels of slicing from the facet harboring the opening hole toward the exit hole localized at the opposite side.

Figure 9A is a representation of MjTET entry hole is lined by three negative patches. One of these patches made up by dimer (colored in green and blue), is highlighted by dashed circle.

Figure 9B: Deeply slice, inside MjTET particle, shows positive patches formed by internal loops. At this entrance, one loop, more precisely that one of blue monomer, is engaged in the positive observed charge and is highlighted by dashed circle. While the second loop of the green monomer is orientated toward the adjacent opening hole (not shown here).

Figure 9C is an observation of the opposite exit pore where three small channels made up by side chain of Arg215 and Phe219 of three monomers, can be observed.

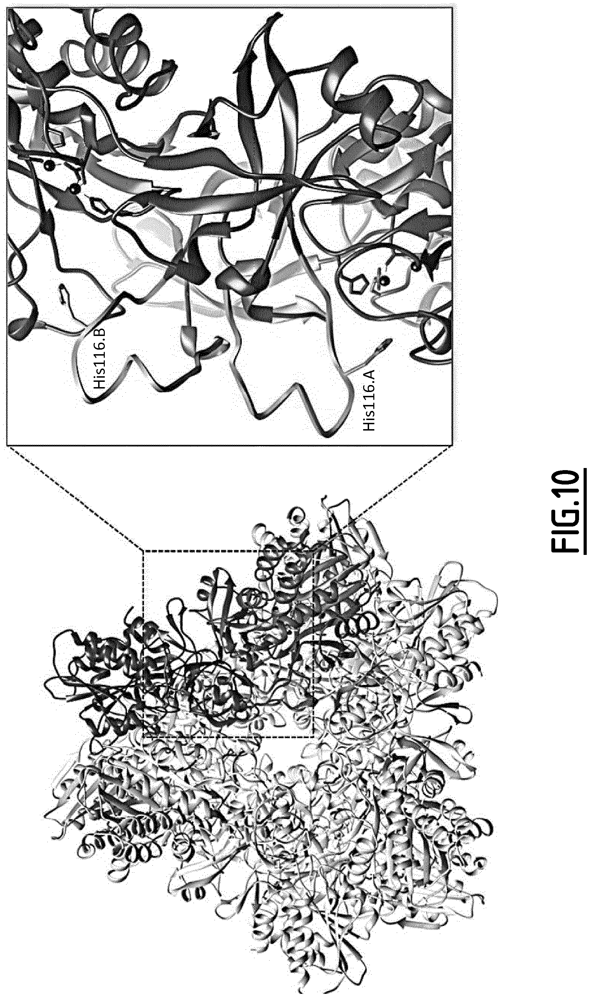

**Figure 10** represents the topological location of two loops carried by two monomers forming the dimeric building block of the tetrahedral particle. Each loop of each monomer (A or B, colored in green and purple, respectively) is inserted near the active site of the adjacent monomer in the same building block. Histidine residue (His116) is orientated in the direction of the active site, where black spheres represent the two $Zn^{2+}$ ions.

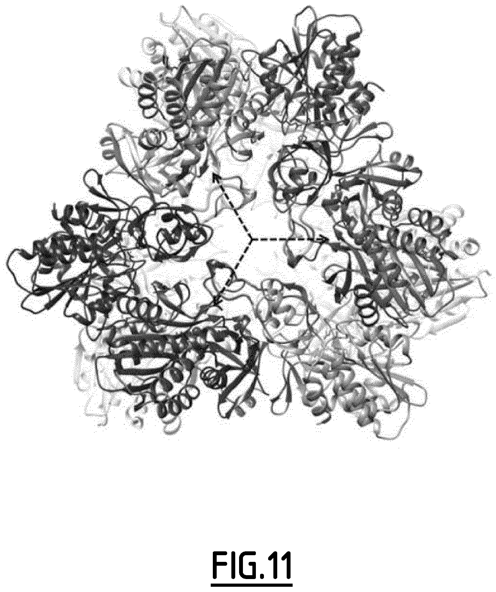

**Figure 11** is a representation of a topological localization of three internal loops behind the opening hole. Each opening hole is built by three dimers, and each dimer monomer participates with two other monomers (of different dimers) in the genesis of the four particle apexes. Here, three apexes are represented with different nuance colors of green, purple and blue. Three internal loops (light green, magenta and cyan) are localized behind the entrance pore; each one is carried by one monomer of a building block. These loops guide peptide toward one of the three active sites, where zinc ions are represented as red spheres, as shown by dashed arrow.

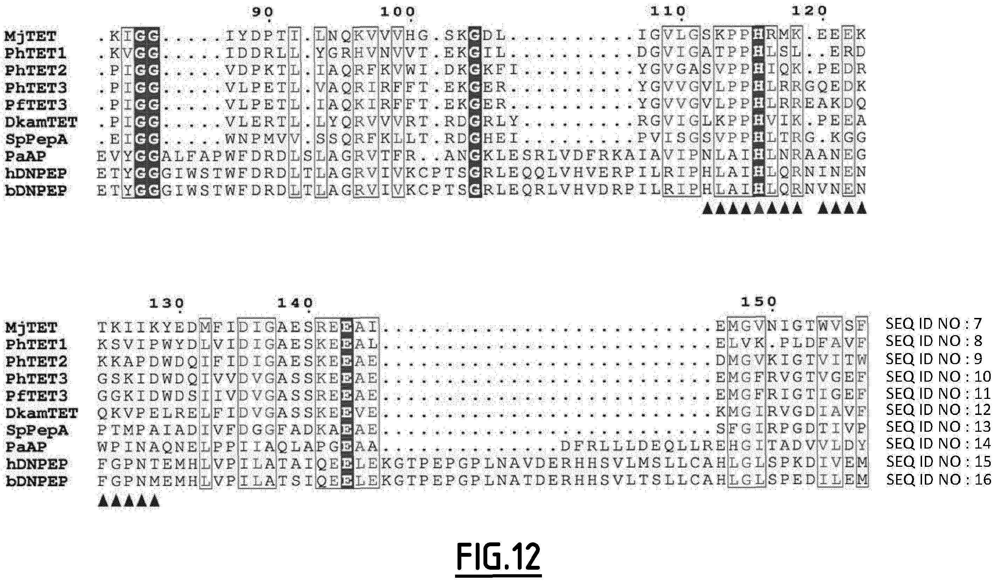

**Figure 12** represents a sequence alignment of M18 and M42 dodecameric aminopeptidases. Internal loop residues are marked by blue triangles according to those of MjTET. This alignment shows conservation of histidine residues (red triangle) amongst M18 and M42 aminopeptidases. PhTET1, PhTET2, PhTET3, PfTET3 and DkamTET are archaeal aminopeptidases expressed by Pyrococcus horikoshii, P. furiosus and Desulfurococcus kamchatkensis, respectively. SpPepA and PaAP are bacterial aminopeptidases expressed by Streptococcus pneumoniae and Pseudomonas aeruginosa, respectively. hDNPEP and bDNPEP are human and bovine aminopeptidases, respectively. MjTET, PhTET1, 2 and 3, PfTET3, DkamTET and SpPepA belong to M42 family, while PaAP, hDNPEP and bDNPEP belong to M18 family according to MEROPS classification. Sequence alignment was done by using ESPript 3.0 server.

**Example 1**

**Experimental procedures**

***Protein expression and purification***

Genes encoding for MjTET and its tagged form MjTET-Nt (with N-terminal $His_6$ tag) were synthesized and cloned by GeneCust Europe in pET-41c and pET28a+ vectors, respectively. The inventors used *Escherichia coli* ArcticExpress (DE3)-RIL strain (Agilent Technologies) for the over-expression of these two recombinant proteins. The cells were grown in 1 L of lysogeny broth (LB) medium containing gentamycin and kanamycine at 50 $\mu$g/ml. Then the cells were incubated with shaking at 37 °C until the $A_{600}$ reached 0.6-1.0, at this stage the induction was carried out by adding isopropyl $\beta$-D-thiogalactopyranoside at final concentration of 0.1 mM and proteins are overexpressed during 7 h at 16 °C. The induced cells were harvested by centrifugation, and the pellets were then conserved at -80 °C until use. These pellets were suspended in 50 ml of 50 mM Tris-HCl, 200 mM NaCl and 0,1 % Triton X-100, pH 7.5, supplemented with 12.5 mg of lysozyme (Euromedex), 2.5 mg of DNase I grade II, 10 mg of RNase, 50 mg of Pefabloc SC (Roche), 500 $\mu$l of 2 M $MgSO_4$ and 50 mM imidazole for cells expressing MjTET-Nt protein. The cell disruption was achieved by sonication in a Branson sonifier at 30 watts for 10 cycles on/off of 30 sec each, at 4 °C. The crude extracts were heated at 85 °C for 20 min and later clarified by centrifugation (JA20 rotor) at 12000 rpm for 1 h at 4 °C. The supernatant was diluted two times with 20 mM Tris-HCl pH 7.5, and loaded on anion exchange column (Resource Q) (GE Healthcare Life Sciences) for MjTET recombinant form. The protein was eluted with a linear NaCl gradient from 190-340 mM in 20 mM Tris-HCl pH 7.5. While MjTET-Nt purification was performed on HiTrap Chelating HP column (GE Healthcare Life Sciences) with 200 and 300 mM imidazole in 20 mM Tris-HCl, 200 mM NaCl, pH 7.5. Exclusion chromatography for the two protein forms was done on Superose 6 column or Superdex 200 column (GE Healthcare Life Sciences) previously equilibrated with 20 mM Tris-HCl, 200 mM NaCl, pH 7.5. The fractions containing protein (38/40 kDa) according to SDS-PAGE were pooled and concentrated using Amicon cell (Millipore) with a molecular mass cutoff of 30 kDa and kept at 4 °C. 3 and 21.5 mg of MjTET and MjTET-Nt were produced from 1 L of culture, respectively.

### Negative Electron Microscopy

MjTET samples, at approximately 0.05 mg/mL in 20 mM Tris-HCl and 200 mM NaCl, pH 7.5, were adsorbed to the clean side of a carbon film on mica, stained with 2 % sodium silicotungstate pH 7.5 and transferred to a 400-mesh copper grid. The images were taken under low dose conditions (<10 e$^-$/Å$^2$) at a magnification of 23 and 49 kX times with defocus values between 1.2 and 2.5 $\mu$m on a Tecnai 12 LaB6 electron microscope at 120 kV accelerating voltage using CCD Camera Gatan Orius 1000.

### Effect of pH on MjTET activity

The effect of pH on MjTET activity was determined in pH range varying from 6 to 11. The buffers used were: PIPES, pH 6-7.5; CHES, pH 8.2-10 and CAPS, pH 10.5-11. All buffers were used at 50 mM concentration in presence of 150 mM KCl, 0.1 mM CoCl$_2$, 4 $\mu$g/mL of MjTET and 5 mM Leu-pNA as substrate. The given pH values are at 75 °C. Incubation was done at 75 °C for 5 min and the quantity of produced pNA was evaluated by measuring the absorbance at 405 nm.

### Effect of metal cations on MjTET activity

The metal cations XCl$_2$ were incubated at 0.1 mM concentration with 4 $\mu$g/mL of MjTET and 5 mM of Leu-pNA in 50 mM CHES, 150 mM KCl, pH 8.5 for 5 min at 75 °C. To assess the effect of metal cations on MjTET activity, the enzymatic reaction was followed as described before.

### Effect of temperature on MjTET activity and stability

Different temperatures ranging from 20 to 90 °C were tested on MjTET activity. Incubation was done in 50 mM CHES, 150 mM KCl, 0.1 mM CoCl$_2$, pH 8.5 in presence of 4 $\mu$g/mL of protein and 5 mM Leu-pNA for 5 min. Absorbance of released pNA was measured at 405 nm.

The effect of temperature on MjTET stability was assayed by incubating the enzyme (160 $\mu$g/ml) at 85 °C. Aliquot of 10 $\mu$l was taken at different time intervals, and the remaining aminopeptidase activity was assayed against 5 mM Leu-pNA in 390 $\mu$l of 50 mM CHES, 150 mM KCl, 0.1 mM CoCl$_2$, pH 8.5 incubated at 85 °C for 5 min. Enzymatic stability of MjTET was evaluated by measuring the quantity of produced pNA at 405 nm.

### Effect of inhibitors on MjTET activity

The inhibitory effect of EDTA, Amastatine and Bestatine was tested on the MjTET enzymatic activity. 4 $\mu$g/mL of MjTET were incubated with each inhibitor separately in the presence of 5 mM Leu-pNA, 50 mM CHES, 150 mM KCl, 0.1 mM CoCl$_2$, pH 8.5 at 85 °C for 5 min. Three replicates (with inhibitor) and two controls (without inhibitor) were assayed for each experimental point.

### Determination of MjTET activity on both synthetic chromogenic, fluorogenic and peptide compounds

The hydrolytic activity of MjTET on synthetic chromogenic and fluorogenic compounds was determined by using different aminoacyl-pNAs and aminoacyl-AMCs through the following procedure. Reactions were initiated by addition of 4 $\mu$g/mL of enzyme to a pre-warmed mixture containing 5 mM of the chromogenic or fluorogenic compound in 50 mM CHES, 150 mM KCl and 0.1 mM CoCl$_2$, pH 8.2, in a total volume of 400 $\mu$l. Incubation was performed at 75 °C for 5 min. The quantity of liberated pNA was evaluated by measuring the absorbance at $\lambda$=405 nm in a JASCO V-630 spectrophotometer, and for AMC by measuring the fluorescence using excitation and emission wavelengths of 360 and 440 nm, respectively in a JASCO FP-8500 spectrofluorometer. Three replicates and two enzyme blanks were assayed for each experimental point. Additional enzymatic studies were performed in order to investigate MjTET capacities to hydrolyze longer substrates (H-Trp-Phe-Tyr-Ser(PO$_3$H$_2$)-Pro-Arg-pNA (SEQ ID NO : 3), H-Met-Trp-Ala-Glu-Asn-Lys (SEQ ID NO:4), H-Tyr-Asp-Thr-Ser-Ile-Val-Gln-Arg (SEQ ID NO:5), H-Asp-Ala-Tyr-Pro-Ser (SEQ ID NO: 6) and H-Glu-Gly-Ile. 2 $\mu$g/ml (final concentration) of MjTET were added to a pre-warmed mixture of 0.75 mM peptide, 50 mM CHES, 150 mM KCl, 0.1 mM $CoCl_2$, pH 8.5. To avoid water evaporation, 20 $\mu$l of mineral oil were added on the top of the total volume. The reaction incubation was done at 85 °C during 6 min. Aliquot of 80 $\mu$l was then removed and added to 220 $\mu$l of 2 % acetonitrile (ACN), 0.1 % trifluoroacetic acid (TFA). Proteins were removed by centrifugation at 13,000 rpm during 15 min. 100 $\mu$l of the supernatant were retrieved and injected on Nova-Pak C18 column, (4 $\mu$m, 3.9 x 300 mm, (Waters)) in a HPLC purifier system (PerkinElmer), equilibrated with 2 % ACN, 0.1% TFA. The elution of peptide products was achieved with a linear ACN gradient (2-33.2 %) and followed by measuring the absorbance at 214 nm. Chromatographic runs were carried out at room temperature.

### *MjTET-Nt crystallization, data collection, structure determination and refinement*

MjTET-Nt was purified as described above and concentrated up to 5.6 mg/ml using an Amicon Ultra 30 kDa cut/off filter. Crystals were grown at 20 °C in Trisodium citrate 0.1 M pH 5.6, Tertbutanol 15 % using the hanging drop vapor diffusion method. MjTET-Nt was mixed with an equal volume of the crystallization solution and crystals grew up to average 80 $\mu$m size in two days. The crystals were cryo-protected with Trisodium citrate 0.1 M pH 5.6, Tertbutanol 15 % supplemented with 2-Methyl-2,4-pentanediol 25 %. The crystals were flash cooled in liquid nitrogen prior to data collection at 100 K. Data set was collected at ESRF beamline ID29. The diffraction extended up to 2.4 Å. Data were indexed and integrated by XDS (Kabsch, 2010 Acta crystallographica. Section D, Biological crystallography 66, 125-132). Integrated intensities were scaled and merged with SCALA and TRUNCATE from the CCP4 programs suite (19). MjTET-Nt

| Structure | MjTET-Nt(-_Zn2) | MjTET-Nt(Zn1_Zn2) |
|---|---|---|
| Beam line | ID29 (ESRF) | ID29 (ESRF) |
| Space group | $P2_13$ | $P2_13$ |
| Unit cell constants (Å) | a=166.54 b= 166.54 c= 166.54 | a=166.15 b=166.15 c=166.15 |
| Resolution range (Å) | 48.08 – 2.40 (2.53 – 2.40) | 47.96 – 2.69 (2.84 – 2.69) |
| Wavelength (Å) | 1.2398 | 1.0332 |
| R-merge (%) | 10.1 (80.9) | 9.2 (72.4) |
| R-pim | 6.2 (49.4) | 4.9 (39.1) |
| CC1/2 | 97.2 (55.5) | 99.7 (72.5) |
| Mean $I/\sigma(I)$ | 6.0 (1.5) | 9.1 (1.6) |
| Completeness (%) | 99.3 (99.1) | 99.2 (96.0) |
| Redundancy | 4.3 (4.2) | 4.9 (5.0) |
| Unique reflections | 59713 (8617) | 42082 (5899) |
| **Refinement:** | | |
| R-work (%) | 18.59 | 17.55 |
| R-free (%) | 22.91 | 23.68 |
| RMSD (angles) | 1.18 | 1.56 |
| RMSD (bonds) | 0.011 | 0.016 |
| **Ramachandran plot:** | | |
| Most favored region (%) | 95.52 | 94.95 |
| Allowed region (%) | 3.73 | 4.53 |
| Outliers (%) | 0.75 | 0.52 |
| **Number of atoms:** | 10898 | 10554 |
| Protein | 10464 | 10494 |
| Water | 430 | 0 |
| Heteroatoms: | 4 | 60 |
| Zinc | 4 | 8 |
| Citrate | 0 | 52 |
| **Mean isotropic B-factors** | 42.80 | 72.45 |
| Protein | | |
| Chain A | 37.92 | 63.62 |
| Chain B | 41.30 | 71.81 |
| Chain C | 46.28 | 77.02 |
| Chain D | 45.57 | 77.29 |
| Water | 43.45 | 0 |
| Zinc | 59.43 | 67.56 |
| Citrate | 0 | 77.37 |

structure was determined by molecular replacement using PHASER MR (20) and PhTET2 as searching model (PDB ID: 1XFO) depleted of side chains. A clear solution was determined with a Translation Function Z-score of 12.4. The asymmetric unit contains four monomers and the space group is cubic $P2_13$. The model was manually completed and improved in COOT prior to refinement with PHENIX (1.10-2155 version) software package (Adams et al., 2011). The model was then optimized through iterative rounds of refinement and model building resulting in final R-work= 0.1859 and R-free= 0.2291 **(Table 1).**

<u>Table 1</u>: **Data collection and refinement statistics of MjTET-Nt(-_Zn$_2$) and MjTET(Zn$_1$_Zn$_2$).** Values in parenthesis correspond for the highest resolution shell.

$$R - merge = \frac{\sum_{hkl} \sum_j |I_{hkl,j} - <I_{hkl}>|}{\sum_{hkl} \sum_j |I_{hkl,j}|}$$

where I$_{hkl, j}$ is the j$^{th}$ intensity measurement of reflection hkl and < I > is the average intensity from multiple observations.

$$R - pim = \frac{\sum_{hkl} \sqrt{\frac{1}{n-1}} \sum_{j=1}^{n} |I_{hkl,j} - <I_{hkl}>|}{\sum_{hkl} \sum_j |I_{hkl,j}|}$$

with I$_{hkl,j}$ is the j$^{th}$ intensity measurements of reflection hkl and < I > is the average intensity from multiple observations. n represents the multiplicity of the measurements.

CC$_{1/2}$= correlation coefficient between random half datasets.

$$R_{work} = \frac{\sum_{hkl} ||F_O| - |F_C||}{\sum_{hkl} |F_O|}$$

for all data except 4% which were used for R$_{free}$ calculation.

During the initial step of refinement, the inventors realized that MjTET-Nt structure was depleted of Zn$^{2+}$ in Site1. Consequently, in order to provide description of MjTET active site, MjTET-Nt crystals were soaked with Zn$^{2+}$ excess solubilized in the cryo-protection solution used above. Data at 2.69 Å resolution were collected at ESRF beamline ID29. Data processing and refinement were conducted as described above, except that the 2.4 Å resolution MjTET-Nt(-_Zn1) model was used as reference model to steer refinement of MjTET(Zn1_Zn2) resulting in final R-work= 0.1755 and R-free= 0.2368 **(Table 1).**

### Cryo-electron microscopy of MjTET dodecamers

Four microliters of sample was applied to 2:1 Quantifoil holey carbon grid (Quantifoil Micro Tools GmbH, Germany) and the grid was frozen in liquid ethane with a Vitrobot Mark II (FEI, the Netherlands). The sample was observed with FEI Polara at 300 kV. Images were recorded on K2 summit direct detector (Gatan Inc., USA) in super resolution counting mode. Movies were recorded at a nominal magnification of 23,000 (0.82 Å/pixel at the camera level) for a total exposure of 4s and 100 ms per frame resulting in 40 frame movies with a total dose of ~40 e$^-$/Å$^2$. 100 movies were manually recorded with Digital Micrograph (Gatan Inc., USA).

Movies were first motion corrected with unblur (Grant, T., and Grigorieff, N. (2015) Elife 4, e06980). Around 2000 particles were picked semi automatically with boxer from the EMAN suite (Ludtke, S. J., et al (1999) J Struct Biol 128, 82-97) and 2D classified in 20 classes in RELION 1.4 (Ludtke, S. J., et al. (1999) J Struct Biol 128, 82-97). The bestdefined 2D class averages were used to generate an initial model using the RICO server imposing tetrahedral symmetry. The resulting low-resolution reconstruction was used as a template for automatic particle picking using the Fast Projection Matching (FPM) method (Estrozi, L. F., and Navaza, J. (2008) J Struct Biol 162, 324-334) as described in (Effantin, G., et al. (2016) PLoS pathogens 12, e1005721). To speed up calculation, the raw micrographs were binned by a factor of 8 (6.56 Å/pixel) and, during template matching, the resolution was limited to 3 nm. For each micrograph, only particles having cross correlation with the reference higher than the mean correlation of all the particles were kept. The output coordinate files from FPM were converted to boxer one (.box) and imported in RELION 1.4 where all subsequent steps were done with two-times binned images (final sampling of 1.64). CTF estimation, particle extraction and preprocessing were done in RELION. The data set was first cleaned by 2D classification. A first 3D auto-refine was then done using the *ab-initio* model obtained with the RICO server imposing tetrahedral symmetry. The obtained 3D model was used as input for 3D classification in five classes. The particles belonging to the classes showing the best resolved features were used to do a final 3D auto-refine. The final reconstruction includes 20000 out of 50000 and has a resolution of 4.1 Å at FSC = 0.143.

### Results

### MJ0555 gene encoded for tetrahedral dodecameric protein

To gain insight into the basic aspects of TET structure and function, the inventors cloned and expressed in *E. coli* MJ0555 gene encoding for the single putative TET peptidase that is present in the genome of *M. jannaschii*. The corresponding recombinant protein was purified with protocols inspired from those developed for the 3 hyperthermophilic TET from *P. horikoshii.* When analyzed by gel filtration chromatography, MJ0555 protein displayed an apparent molecular mass similar to one determined for the homologous TET dodecameric proteins from *P. horikoshii* and *P. furiosus.* Negative stain electron microscopy images of MJ0555 samples showed typical hollow-tetrahedral dodecameric particles with dimensions similar to those previously determined for the different M42 TET peptidases and MJ0555 was therefore named MjTET **(Figure 1).**

### *Influence of pH, metal ions, and temperature on leucine aminopeptidase activity of MjTET*

The sequence alignment of MjTET with other TET peptidases from hyperthermophilic archaea revealed ~45% sequence identity with PhTET2 that was defined as leucine metallo-aminopeptidase (LAP). The optimal conditions for MjTET activity were therefore tested using Leu-pNA. To assess for the metallic dependent behavior of MjTET, the inventors tested several inhibitors of M42 metallopeptidases. Under optimal conditions of MjTET activity (see bellow), adding 5 mM EDTA to the reaction volume completely inhibited the enzyme, thus confirming the metallic dependence of MjTET. Moreover the MjTET LAP activity was completely inhibited by 0.2 mM amastatine and 1 mM bestatine, respectively aminopeptidase and leucyl aminopeptidase inhibitors.

The inventors investigated the effect of pH on MjTET LAP activity. **Figure 2A** shows that MjTET maintained high percentage of activity (between 88 to 100%) over a wide range of pH, from neutral (pH 7) to alkaline (pH 10). Moreover it maintains more than 50% of its activity in acidic and alkaline conditions such as pH 6 and 10.5. Thus as PhTET2, MjTET is an aminopeptidase that can work on broad range of pH conditions (between 6 and 9, for PhTET2). As different divalent metal ions have been found to modulate the activity of aminopeptidases, the inventors tested the effect of several metals on MjTET activity. The data presented on **Figure 2B** showed that the increase of LAP activity occurs only in the presence cobalt ions. By contrast, a significant inhibition was observed when 0.1 mM $ZnCl_2$ was added to the reaction mixture. Thus, MjTET can be defined as cobalt-activated enzyme.

The temperature dependence of MjTET activity was determined. As shown in **Figure 2C,** LAP activity was found to be maximal at 85 °C, corresponding to the optimal growth temperature of *M. jannaschii* species. When incubated at this temperature, MjTET showed a half-life of eight hours. No activity was detected in mesophilic conditions under 50 °C.

### *MjTET is a leucine aminopeptidase with extended substrate specificity*

A common hallmark to M42 TET aminopeptidases studied so far is their marked preference for a subset of amino acids. Hence, in *P. horikoshii,* PhTET1 exhibits narrow selectivity toward glutamyl and aspartyl residues while PhTET2 is a leucyl aminopeptidase with broad activity against neutral amino acids and PhTET3 is a lysyl aminopeptidase with clear preference for positively charged residues (14-16). In order to determine MjTET function, its amino acid selectivity was investigated using chromogenic and fluorogenic monoacyl substrates. As shown in **Figure 3,** the enzyme acted on large number of aminopeptidase substrates. The optimal amidolytic activity was observed against Leu-pNA, thus allowing classifying MjTET as leucine aminopeptidase. Interestingly, MjTET also displayed significant amidolytic activities, principally against hydrophobic residues such as phenylalanine, methionine and tyrosine. Reduced activities were also detected in the presence of polar and positively charged residues. Aspartate and glutamate, cysteine and proline were the only residues toward which no activity was detected. Finally, no activity was detected against blocked residues and residues in D-stereo configuration, as shown for Ac-Leu-pNA and D-Leu-pNA substrates, respectively. M42 aminopeptidases displayed poor amidolytic activity against aromatic substrates such as phenylalanine and tyrosine. PhTET2 was found to exhibit weak activity against aromatic amino acids, however, as shown in **Figure 4,** MjTET enzymatic activities appeared to be more efficient notably against phenylalanine. In addition to the broad activity spectrum, this represents another noticeable difference between MjTET and PhTET2.

The exopeptidase activities and specificities can be different in peptide context, compared to the one measured on monoacyl compounds. To test if MjTET maintains broad amino acid substrate specificity as well as the ability to cleave aromatic residues in peptide context, its action was studied on different substrates containing 6 to 8 residues. After incubation with MjTET enzyme, the peptide composition of the reaction mixture was investigated at different time points by reverse phase HPLC. **Figure 5A** shows the hydrolysis profile of the H-Trp-Phe-Tyr-Ser($PO_3H_2$)-Pro-Arg-pNA peptide. After 5 minutes, the original peptide substrate was almost hydrolyzed and four peptide products were detected, thus confirming that the enzyme displays broad aminopeptidase activity, even on peptides containing aromatic residues. This was confirmed with other substrates: H-Asp-Ala-Tyr-Pro-Ser **(Figure 5B),** H-Tyr-Asp-Thr-Ser-Ile-Val-Gln-Arg, H-Met-Trp-Ala-Glu-Asn-Lys, and H-Glu-Gly-Ile (data not shown). Interestingly, these peptides also contained Asp and Glu residues. Since no activity was measured against Asp-pNA and Glu-pNA, it can be inferred that, these negatively charged amino acids can also be cleaved by MjTET in peptide context, indicating that C-terminal end is required for better fitting of the P1 amino acid inside "S1" specificity pocket of the enzyme. Taken together these experiments showed that, compared to other M42 aminopeptidases, MjTET is characterized by unusual broad substrate specificity.

*Topology of MjTET enzyme determined by X-ray crystallography*

Crystal structure of MjTET-Nt was resolved at 2.4 Å by molecular replacement using PhTET2 monomer structure (PDB ID: 1XFO) (27) as search probe. The asymmetric unit of the crystal encloses four monomers, each of ~40 kDa and made up of 366 amino acids. The monomer had typical structure of M42 aminopeptidases forming TET dodecamers. Comparison of the dimerization and oligomerization interfaces of MjTET and PhTET2, by using PISA server (http://www.ebi.ac.uk/pdbe/pisa/), revealed that they are very similar and involve the same monomer contacts with nearly the same number of hydrogen bonds and salt bridges.

MjTET monomer can be divided in two $\alpha/\beta$ globular domains connected by two loops (residues 63-66 and 158-162) **(Figure 6A).** The bigger domain is the catalytic one formed by residues 1-66 and 158-349 and contains the N- and C-terminal ends. The smaller one is the dimerization domain (also named dimerization domain I) formed by residues 67-157. No electron density was observed for His-tag Nt neither for 115-124 residues. The catalytic domain contains central eight-stranded $\beta$-sheet surrounded by seven $\alpha$-helices and two $3_{10}$ helices. The two $\beta$-strands located on each side of this $\beta$-sheet are antiparallel and shorter than the central four $\beta$-strands, which are much longer and parallel. Additionally, three-stranded antiparallel $\beta$-sheet (residues 162-171 and 325-328) is positioned on the surface of the catalytic domain and can be named dimerization domain II. The dimerization domain I slightly exhibits cylindrical $\beta$-barrel shape made up of six antiparallel $\beta$-strands and two $\alpha$-helices and one $3_{10}$ helix.

*MjTET binuclear active sites*

The cleft located between the catalytic and dimerization (I) domains harbors the binuclear active site of the metallo-aminopeptidase. In untreated MjTET-Nt crystals, only one metal ion per monomer of protein was detectable (MjTET-Nt(-_Zn2)). The second exchangeable metal (M1) was detected after soaking MjTET-Nt crystals with excess of zinc ions during cryoprotection step. In this condition, two $Zn^{2+}$ ions are modeled in the active site of the enzyme (MjTET-Nt(Zn1_Zn2)). $Zn_1^{+2}$ has a trigonal bipyramidal geometry and is coordinated by Asp175O$^{\delta2}$, Glu208O$^{\epsilon1,\epsilon2}$, His321N$^{\epsilon2}$ and Citrate O$^{\beta2}$. However, $Zn_2^{+2}$ is tetrahedrally coordinated by His62 N$^{\epsilon2}$, Asp175O$^{\delta1}$ and Asp230O$^{\delta1,\delta2}$ **(Figure 6B).** The distance between the metal ions is ~3.87 Å comparable to that one of all other M42 TET peptidases and the leucyl aminopeptidase AAP of *Vibrio proteolyticus* (formerly known as *Aeromonas proteolytica)* used as prototype to describe the enzymatic mechanism of peptide bond hydrolysis by binuclear aminopeptidase (28).

*Specificity pocket properties explain MjTET broad substrate specificities*

Behind the active site is located the specificity pocket "S1" that was suggested to receive the side chain of the residue that will be cleaved (29). A mainly large and hydrophobic specificity pocket (residues 231-233, 254, 256, 259-262, 264, 289, 291, 293-296, 320) was found in MjTET monomer, which is in agreement with the preference of this aminopeptidase to hydrolyze hydrophobic residues such as leucine and methionine as described above. Interestingly, this pocket is characterized by the presence of glycine residues (Gly256, 262, 264, 291, 293 and 294) that may provide important flexibility to this region, thus allowing the reception of large side chains such as those of aromatic residues especially phenylalanine and tyrosine. Furthermore, negatively charged amino acids, such as Asp259 and Glu289, also present in the "S1" pocket could interact with the positive side chains of histidine, lysine and arginine for potential cleavage by MjTET **(Figure 7).**

*Combining crystallographic and cryo-EM data allowed the determination of MjTET complete atomic model*

Previous structural studies showed that, despite their similar architectures, the different types of TET complexes showed differences in their secondary and tertiary structures as well as pores and channels dimensions (3). Noticeable changes were also detected in the surface charge properties of the different TET edifices, in particular with respect to the electrostatic charge distribution in the specificity pocket. These differences were proposed to be responsible for the contrasted substrate specificities between the different TET enzymes and allowed to propose models for peptide trafficking inside the particle (13,14,16,30). However, there is a lack of structural information on the TET particle interior, apparently due to flexible regions that could not be completely resolved by X-ray crystallography, which preclude validating the proposed models. In this work, the inventors combined X-ray crystallography and cryo-EM experiments to provide better insights into the general internal organization of TET particles.

*Internal structural organization of MjTET revealed by cryo-EM*

Intriguingly, electron density was usually absent for the loops harbored by the dimerization domain (I) in crystal structures of TET complexes, especially those belonging to M42 family. This observation indicated the mobile behavior of these loops and may suggest an important role in substrate trafficking. In MjTET cryo-EM structure, electron densities of the twelve loops (residues 114-124) are clearly visible allowing us to build the X-ray structure missing parts **(Figure 8A).** In fact, to get the atomic structure of X-ray missed loops, the inventors fitted the X-ray structure of MjTET dimer (building block) in cryo-EM map resolved at 4.1 Å, wherein the inventors built up the internal loop residues (115-124) of the dimers, by using Coot software (31). Then, the inventors generated automatically the complete tetrahedral particle for which the inventors checked the position of all constructed internal loops. The analysis of complete particle structure achieved by combining crystallography and cryo-EM, revealed new features regarding internal organization of tetrahedron. Indeed, the inventors showed here that MjTET entire loops established internal bulk inside the dodecameric macromolecule **(Figure 8B).** MjTET electrostatic potential showed strong positive charge engendered by these internal loops. They appeared to disrupt peptide trafficking inside the particle. Interestingly, these positive patches are located behind negatively charged one created by acidic residues of three building blocks forming the opening pore of the particle **(Figure 9).** These negative charges were described previously to captivate positively charged N-termini of peptide substrate. The location of internal positively charged loops behind the negative patches might avoid peptide navigation from entry pore to the exit hole located on opposite side of the tetrahedron. Furthermore, the organization of the dynamic loops in the same building block, showed that each loop extended near the active site of adjacent monomer, with a peculiar orientation of His116 toward this neighboring site **(Figure 10).** Together, the newly defined positive network that obstructed the internal cavity of MjTET particle and the loop orientation toward active site provide a significantly shorter route from the outside of TET to one of the three catalytic chambers present near the entry hole compared to the alternative route that crosses the central cavity to catalytic chamber situated at the opposite side of the tetrahedron **(Figure 11).**

## Discussion

### *Single TET peptidase from Methanocaldococcus jannaschii is a non-specialized peptidasome*

Genomic survey and functional characterization indicate that in prokaryotic cells the number of TET proteins can vary from one to up to four different versions. In the deep-sea hyperthermophilic archaeon *P. horikoshii,* three different versions of TET complexes having contrasted substrate preferences have been described (13,15,16,30). According to MEROPS classification, *M. jannaschii,* another hyperthermophilic archaeon isolated from a natural environment similar to the one of *P. horikoshii,* contains only one gene encoding for a putative M42 family TET enzyme of MH clan. In this work, the inventors showed that the corresponding recombinant protein, called here MjTET, is indeed a TET aminopeptidase. The search for its physiological function using monoacyl and peptide substrates revealed the enzyme is a leucine aminopeptidase able to cleave hydrophobic, neutral as well as aromatic and positively charged residues such as phenylalanine, tyrosine, lysine and histidine. MjTET can therefore be qualified as non-specialized peptidasome. Unlike *P. horikoshii, M. jannaschii* is an autotrophic organism that does not rely on the hydrolysis of external peptide to support its metabolism. Thus, the inventors can speculate that the presence of single TET system, "a priori" less efficient than the multiple specialized TET enzymes found in Thermococcales, could be related to autotrophy.

PhTET2 was also described as leucine aminopeptidase. It shares ~45% of sequence identity with MjTET. However, its specificity range is not as wide as the one of MjTET and it is devoid of hydrolytic activity toward N-terminal phenylalanine residues. Therefore, the comparison of the two enzyme 3D structures can help to determine the structural determinants responsible for the remarkable MjTET polyvalence. This analysis showed that the "S1" specificity pocket of MjTET enzyme is slightly larger than PhTET2 one, and is enriched by glycine residues that are not present in other specialized TET enzymes. Compared to PhTET2, MjTET "S1" pocket contains three additional residues and three glycine residues that could confer more flexibility thus allowing accommodating phenylalanine and tyrosine large side chains. Compared to MjTET, the reduce activity of PhTET2 against positively charged substrates could be explained by the presence of Lys261 (in reference to PhTET2 sequence) that can disrupt positive side chain fitting in "S1" pocket. In peptide context, MjTET also displayed significant cleavage capacity of acidic residues. Colombo et *al.* revealed the presence of third metal ion in the specificity pocket of PfTET3, a lysyl aminopeptidase of *P. furiosus* (26). This cation is coordinated by Thr232 and Glu281 residues and facilitate the accommodation of negative charged side chain of substrates in "S1" pocket and subsequently their degradation (26). In similar fashion, the "S1" pocket of MjTET encloses Thr232 and Glu289, which are localized in the same position as the former ones, allowing the possibility to receive a third metal, thus extending MjTET cleavage capacity to acidic residues.

### *TET system consists in 4 independent peptide-processing modules*

The cryo-EM experiments allowed the edification of the twelve internal dynamic loops carried by the dimerization domains inside the M42 MjTET particle cavity. The loop positions with respect to the active sites, entry holes and their topology within the central cavity suggest that they are of pivotal importance for the functioning of the TET dodecamers. In particular, the cryo-EM map revealed that some internal loops residues, particularly His116, are orientated toward the binuclear active site. Sequence alignments reveal a strict conservation of His116 amongst M18 and M42 aminopeptidases exhibiting tetrahedral architecture **(Figure 12)**. In eukaryotic M18, His170 of hDNPEP (human aspartyl aminopeptidase) and His166 of bDNPEP (bovine aspartyl aminopeptidase) are localized near the active site and were proposed to be involved in substrate binding and in the stabilisation of tetrahedral intermediate of enzymatique reaction (8,9). Moreover, the His170Phe mutation completely abolished the enzymatic activity of hDNPEP (32). Thus, in M42 TET, histidine residue located at the internal loop extremity is suggested to play important role in peptides hydrolysis. Interestingly in cryo-EM structure, the distance that separates the His116 from the active site is much longer than the one measured in the crystal structures of M18 family TET (characterized by longer amino acid sequence than MjTET one). This may reflect a dynamic movement of MjTET internal loops to be closed to the active site. Thus, this motion allows the orientation of N-ter polypeptide end toward the binuclear catalytic site where hydrolysis will occur.

The position of the internal loops as determined the MjTET particle leads to drastically change our vision regarding the peptide navigation pathway within M42 family TET particles. The twelve internal loops of MjTET built in the electron density of cryo-EM map obstructed the internal cavity. They define a strong positively charged cluster generated by Lys113, His116, Arg117, Lys119, Lys123, Lys125 and Lys128 residues. These features would prevent polypeptide substrates to cross the particle from one entry hole to the opposite apex where three active sites are present, as the inventors proposed before for the three TET from *P. horikoshii.* Thus, another route for peptide navigation must be considered. The analysis of MjTET surface electrostatic potential showed three well-defined negative patches located at the entry hole of the tetrahedron. The comparison of the electrostatic potential of the different thermophilic M42 family TET complexes revealed that this feature is well conserved. In MjTET, each acidic patch is made up by six acidic amino acids, Asp74-75, Glu243, Asp244 and Asp248 of each dimer loop encompassing the large opening of the particle. The inventors noticed that these loops situated at the entrance hole and which harbored precedent negatively charged residues are located in front the internal positive loops at a correct distance and position allowing their interaction. Therefore, the function of the entry loops may be polypeptide capture in such a manner that the substrate N-terminal will be pre-orientated toward the internal cavity and one of the twelve internal positively charged loop devoted to substrate guidance and positioning in the catalytic chamber.

In conclusion, our results indicated that TET combines four peptide-processing modules each consisting in one entry hole in connection with three adjacent catalytic chambers. Three structural loops form a chain to guide the substrate N-termini toward one of three active sites of three adjacent catalytic chambers. The building of these modules also involves three other loops that participate to another functional module, which underline the functional relevance of TET dodecamerization. In this new model, and unlike to the previously proposed one, the four tetrahedron apexes could process simultaneously, and in an independent manner, at least four peptides while avoiding peptide congestion inside the particle.

## Example 2 - **Effect of the temperature**

In order to evaluate the effect of the temperature on the activity of MjTET, the inventors evaluated the relative amount of intact peptides further to an incubation with the TET protein at different temperatures.

The inventors have measured the relative amount of intact peptide further to an incubation with MjTET.

MjTET activity was measured on the following peptide : YFLMNENRYTSWNSE (SEQ ID NO: 2). The inventors evaluated the time necessary to hydrolyse 50% of the peptide at different tempertaures

Results are shown in the following table :

| Temperature (°C) | Time (h) |
|---|---|
| 80 | 0,25 |
| 70 | 0,6 |
| 60 | 1 |
| 50 | 1,3 |
| 40 | 3,9 |
| 30 | 55 |
| 20 | 75 |

Activity of TET proteins on peptides having more than 3 amino acids residues at temperatures below 80°C was never tested. The inventors demonstrate that the peptidase activity on long peptides (15 amino acids) is similar to the activity on dipeptides. Compared to the other TET proteins, MjTET is not very active at 40°C (0.5 h for PhTET2 compared to 3,9 h for MjTET at 40°C).

**Claims**

1. Use of a TET protein as a N-terminus aromatic amino acid residues specific exopeptidase, said TET protein consisting of the amino acid sequence as set forth in SEQ ID NO: 1,
or any homologous protein derived from said TET protein as set forth in SEQ ID NO: 1 by substitution, addition or deletion of at least one amino acid, provided that the derived protein retains at least 70 % of identity with the amino acid sequence as set forth in SEQ ID NO: 1, and said derived protein retaining a N-terminus aromatic amino acid residues specific exopeptidase activity.

2. Use of at least a TET protein harboring at least a N-terminus aromatic amino acid residues specific exopeptidase activity, for the modification of all or part of the polypeptide content of a substrate comprising peptides, polypeptides and/or proteins harbouring an N-terminus aromatic amino acid residue, said at least a TET protein consisting of the amino acid sequence as set forth in SEQ ID NO: 1,
or any homologous protein derived from said at least a TET protein as set forth in SEQ ID NO: 1 by substitution, addition or deletion of at least one amino acid, provided that the derived protein retains at least 70 % of identity with the amino acid sequence as set forth in SEQ ID NO: 1, and said derived protein retaining a N-terminus aromatic amino acid residues specific exopeptidase activity.

3. Use according to claim 1 or 2, wherein said a TET protein or said derived protein originates from an extremophile microorganism belonging to the *Methanococcales* order.

4. Use according to anyone of claims 1 to 3, wherein said extremophile microorganism is *Methanocaldococcus jannaschii.*

5. Use according to anyone of claims 2 to 4, wherein peptides, polypeptides and/or proteins of said substrate are obtained from food, chemical and health industries, or from biomass.

6. Method for degrading, from the N-terminus part, a polypeptide harboring an aromatic residue at its N-terminal part, said method comprising a step of contacting said polypeptide harboring an aromatic residue at its N-terminal part with

    - at least a TET protein harboring at least a N-terminus aromatic amino acid residues specific exopeptidase activity, said TET protein consisting of the amino acid sequence as set forth in SEQ ID NO: 1,
    - or any homologous protein derived from said TET protein as set forth in SEQ ID NO: 1 by substitution, addition or deletion of at least one amino acid, provided that the derived protein retains at least 70 % of identity with the amino acid sequence as set forth in SEQ ID NO: 1, and said derived protein retaining a N-terminus aromatic amino acid residues specific exopeptidase activity,

    and possibly a step of recovering the resulting N-terminal aromatic amino acid residue free peptides.

7. Method for modifying all or part of the polypeptide content of a substrate comprising peptides, polypeptides and/or proteins, said substrate comprising a polypeptide harboring an aromatic residue at its N-terminal part, said method comprising a step of contacting said substrate and degrading, from the N-terminus part, said polypeptide harboring an aromatic residue at its N-terminal part with

    - at least a TET protein harboring at least a N-terminus aromatic amino acid residues specific exopeptidase activity, said TET protein consisting of the amino acid sequence as set forth in SEQ ID NO: 1,
    - or any homologous protein derived from said TET protein as set forth in SEQ ID NO: 1 by substitution, addition or deletion of at least one amino acid, provided that the derived protein retains at least 70 % of identity with the amino acid sequence as set forth in SEQ ID NO: 1, and said derived protein retaining a N-terminus aromatic amino acid residues specific exopeptidase activity,

    and possibly a step of recovering the modified polypeptide content of a substrate.

8. Method according to claim 6 or 7, wherein said step of contacting is carried out at a pH range varying from 6.5 to 10.

9. Method according to anyone of claims 6 to 8, wherein said at least a TET protein or said derived protein is immobilized on a solid support, preferably on a filter cartridge.

10. Method according to anyone of claims 6 to 8, wherein said at least a TET protein or said derived protein is expressed in a mesophilic host, from a plasmid, and then purified from cell lysates by a thermal denaturation step of the host proteins.

11. Use of a solid support for the modification of all or part of the polypeptide content of a substrate comprising peptides, polypeptides and/or proteins, wherein said substrate comprises a polypeptide harboring an aromatic residue at its N-terminal part, wherein said modification comprises degrading, from the N-terminus part, said polypeptide harboring an aromatic residue at its N-terminal part and wherein is immobilized on said solid support:

    - at least a TET protein harboring at least a N-terminus aromatic amino acid residues specific exopeptidase activity, said TET protein consisting of the amino acid sequence as set forth in SEQ ID NO: 1,
    - or any homologous protein derived from said TET protein as set forth in SEQ ID NO: 1 by substitution, addition or deletion of at least one amino acid, provided that the derived protein retains at least 70 % of identity with the amino acid sequence as set forth in SEQ ID NO: 1, and said derived protein retaining a N-terminus aromatic amino acid residues specific exopeptidase activity.

12. Use according to claim 11, wherein said solid support is a filter cartridge, silica or magnetic beads.

13. Use according to claim 11 or 12, wherein peptides, polypeptides and/or proteins of said substrate are obtained from food, chemical and health industries, or from biomass.

**Patentansprüche**

1. Verwendung eines TET-Proteins als eine Exopeptidase, die spezifisch N-terminale aromatische Aminosäurereste erkennt, wobei das TET-Protein aus der Aminosäuresequenz gemäß SEQ ID Nr. 1 besteht, oder aus einem homologen Protein, das von dem TET-Protein gemäß SEQ ID Nr. 1 durch Substitution, Addition oder Deletion von mindestens einer Aminosäure abgeleitet ist, vorausgesetzt, dass das abgeleitete Protein mindestens 70 % Identität mit der Aminosäuresequenz gemäß SEQ ID Nr. 1 aufweist und das abgeleitete Protein eine Exopeptidaseaktivität beibehält, die spezifisch für N-terminale aromatische Aminosäurereste ist.

2. Verwendung von mindestens einem TET-Protein, das mindestens eine Exopeptidaseaktivität spezifisch für N-terminale aromatische Aminosäurereste aufweist, zur Modifizierung des gesamten oder eines Teils des Polypeptidgehalts eines Substrats, das Peptide, Polypeptide und/oder Proteine mit einem N-terminalen aromatischen Aminosäurerest umfast, wobei das mindestens eine TET-Protein aus der Aminosäuresequenz gemäß SEQ ID Nr. 1 besteht, oder aus einem homologen Protein, das von dem mindestens einen TET-Protein gemäß SEQ ID Nr. 1 durch Substitution, Addition oder Deletion von mindestens einer Aminosäure abgeleitet ist, vorausgesetzt, dass das abgeleitete Protein mindestens 70 % Identität mit der Aminosäuresequenz gemäß SEQ ID Nr. 1 aufweist und das abgeleitete Protein eine Exopeptidaseaktivität beibehält, die spezifisch für N-terminale aromatische Aminosäurereste ist.

3. Verwendung gemäß Anspruch 1 oder 2, wobei das TET-Protein oder das abgeleitete Protein von einem extremophilen Mikroorganismus der Ordnung Methanococcales stammt.

4. Verwendung gemäß einem der Ansprüche 1 bis 3, wobei der extremophile Mikroorganismus *Methanocaldococcus jannaschii* ist.

5. Verwendung gemäß einem der Ansprüche 2 bis 4, wobei die Peptide, Polypeptide und/oder Proteine des Substrats aus der Lebensmittel-, Chemie- und Gesundheitsindustrie oder aus Biomasse stammen.

6. Verfahren zum Abbau eines Polypeptids mit einem aromatischen Rest am N-terminalen Ende, ausgehend vom N-Terminus, umfassend den Schritt des Kontakts des Polypeptids mit aromatischem Rest am N-Terminus mit

- mindestens einem TET-Protein mit mindestens einer Exopeptidaseaktivität spezifisch für N-terminale aromatische Aminosäurereste, wobei das TET-Protein aus der Aminosäuresequenz gemäß SEQ ID Nr. 1 besteht,
- oder einem homologen Protein, das durch Substitution, Addition oder Deletion von mindestens einer Aminosäure aus dem TET-Protein gemäß SEQ ID Nr. 1 abgeleitet ist, vorausgesetzt, dass das abgeleitete Protein mindestens 70 % Identität mit der Aminosäuresequenz gemäß SEQ ID Nr. 1 aufweist und eine Exopeptidaseaktivität beibehält, die spezifisch für N-terminale aromatische Aminosäurereste ist,

und ggf. einen Schritt zum Zurückgewinnen der resultierenden Peptide ohne N-terminalen aromatischen Aminosäurerest.

7. Verfahren zur Modifizierung des gesamten oder eines Teils des Polypeptidgehalts eines Substrats, das Peptide, Polypeptide und/oder Proteine enthält, wobei das Substrat ein Polypeptid mit einem aromatischen Rest am N-terminalen Ende enthält, umfassend den Schritt des Kontakts und des Abbaus des Polypeptids mit einem aromatischen Rest am N-terminalen Ende ausgehend vom N-Terminus mit

- mindestens einem TET-Protein mit mindestens einer Exopeptidaseaktivität spezifisch für N-terminale aromatische Aminosäurereste, wobei das TET-Protein aus der Aminosäuresequenz gemäß SEQ ID Nr. 1 besteht,
- oder einem homologen Protein, das aus dem TET-Protein gemäß SEQ ID Nr. 1 durch Substitution, Addition oder Deletion von mindestens einer Aminosäure abgeleitet ist, vorausgesetzt, dass das abgeleitete Protein mindestens 70 % Identität mit der Aminosäuresequenz gemäß SEQ ID Nr. 1 aufweist und eine Exopeptidaseaktivität beibehält, die spezifisch für N-terminale aromatische Aminosäurereste ist,

und ggf. einen Schritt zum Zurückgewinnen des modifizierten Polypeptidgehalts des Substrats.

8. Verfahren nach Anspruch 6 oder 7, wobei der Schritt des Kontakts im pH-Bereich von 6,5 bis 10 durchgeführt wird.

9. Verfahren nach einem der Ansprüche 6 bis 8, wobei das mindestens eine TET-Protein oder das abgeleitete Protein auf einem festen Träger immobilisiert ist, vorzugsweise auf einer Filterkartusche.

10. Verfahren nach einem der Ansprüche 6 bis 8, wobei das mindestens eine TET-Protein oder das abgeleitete Protein in einem mesophilen Wirt aus einem Plasmid exprimiert und anschließend durch einen Schritt der thermischen Denaturierung der Wirtsproteine aus Zelllysaten gereinigt wird.

11. Verwendung eines festen Trägers zur Modifizierung des gesamten oder eines Teils des Polypeptidgehalts eines Substrats, das Peptide, Polypeptide und/oder Proteine umfasst, wobei das Substrat ein Polypeptid mit einem aromatischen Rest am N-terminalen Ende umfasst, wobei die Modifikation den Abbau dieses Polypeptids ausgehend vom N-Terminus umfasst, und wobei auf dem festen Träger immobilisiert ist:

- mindestens ein TET-Protein mit mindestens einer Exopeptidaseaktivität spezifisch für N-terminale aromatische Aminosäurereste, wobei das TET-Protein aus der Aminosäuresequenz gemäß SEQ ID Nr. 1 besteht,
- oder ein homologes Protein, das durch Substitution, Addition oder Deletion von mindestens einer Aminosäure aus dem TET-Protein gemäß SEQ ID Nr. 1 abgeleitet ist, vorausgesetzt, dass das abgeleitete Protein mindestens 70 % Identität mit der Aminosäuresequenz gemäß SEQ ID Nr. 1 aufweist und eine Exopeptidaseaktivität beibehält, die spezifisch für N-terminale aromatische Aminosäurereste ist.

12. Verwendung gemäß Anspruch 11, wobei der feste Träger eine Filterkartusche, Silikaperlen oder magnetische Partikel ist.

13. Verwendung gemäß Anspruch 11 oder 12, wobei die Peptide, Polypeptide und/oder Proteine des Substrats aus der Lebensmittel-, Chemie- und Gesundheitsindustrie oder aus Biomasse stammen.

## Revendications

1. Utilisation d'une protéine TET en tant qu'exopeptidase spécifique des résidus d'acides aminés aromatiques à l'extrémité N-terminale, ladite protéine TET consistant en la séquence d'acides aminés telle que définie dans la SEQ ID NO : 1,
ou toute protéine homologue dérivée de ladite protéine TET telle que définie dans la SEQ ID NO : 1 par substitution, ajout ou suppression d'au moins un acide aminé, à condition que la protéine dérivée conserve au moins 70 % d'identité avec la séquence d'acides aminés telle que définie dans la SEQ ID NO : 1, et que ladite protéine dérivée conserve une activité exopeptidase spécifique des résidus d'acides aminés aromatiques à l'extrémité N-terminale.

2. Utilisation d'au moins une protéine TET présentant au moins une activité exopeptidase spécifique des résidus d'acides aminés aromatiques à l'extrémité N-terminale, pour la modification de tout ou partie du contenu polypeptidique d'un substrat comprenant des peptides, des polypeptides et/ou des protéines présentant un résidu d'acide aminé aromatique à l'extrémité N-terminale, ladite au moins une protéine TET consistant en la séquence d'acides aminés telle que définie dans SEQ ID NO : 1,

ou toute protéine homologue dérivée de ladite protéine TET telle que définie dans SEQ ID NO : 1 par substitution, ajout ou suppression d'au moins un acide aminé, à condition que la protéine dérivée conserve au moins 70 % d'identité avec la séquence d'acides aminés telle que définie dans SEQ ID NO : 1, et que ladite protéine dérivée conserve une activité exopeptidase spécifique des résidus d'acides aminés aromatiques à l'extrémité N-terminale.

3. Utilisation selon la revendication 1 ou 2, dans laquelle ladite protéine TET ou ladite protéine dérivée provient d'un micro-organisme extrêmophile appartenant à l'ordre des *méthanococcales.*

4. Utilisation selon l'une des revendications 1 à 3, dans laquelle ledit micro-organisme extrémophile est *Methanocaldococcus jannaschii.*

5. Utilisation selon l'une des revendications 2 à 4, dans laquelle les peptides, polypeptides et/ou protéines dudit substrat sont obtenus à partir des industries alimentaires, chimiques et de la santé, ou à partir de biomasse.

6. Méthode de dégradation, à partir de la partie N-terminale, d'un polypeptide ayant un résidu aromatique à sa partie N-terminale, ladite méthode comprenant une étape de mise en contact dudit polypeptide ayant un résidu aromatique à sa partie N-terminale avec

- au moins une protéine TET ayant une activité exopeptidase spécifique des résidus d'acides aminés aromatiques à l'extrémité N-terminale, ladite protéine TET consistant en la séquence d'acides aminés telle que définie dans SEQ ID NO : 1,
- ou toute protéine homologue dérivée de ladite protéine TET telle que définie dans SEQ ID NO : 1 par substitution, ajout ou suppression d'au moins un acide aminé, à condition que la protéine dérivée conserve au moins 70 % d'identité avec la séquence d'acides aminés telle que définie dans SEQ ID NO : 1, et que ladite protéine dérivée conserve une activité exopeptidase spécifique des résidus d'acides aminés aromatiques à l'extrémité N-terminale,

et éventuellement une étape de récupération des peptides sans résidus d'acides aminés aromatiques N-terminaux obtenus.

7. Méthode de modification de tout ou partie du contenu polypeptidique d'un substrat comprenant des peptides, des polypeptides et/ou des protéines, ledit substrat comprenant un polypeptide ayant un résidu aromatique dans sa partie N-terminale, ladite méthode comprenant une étape de mise en contact dudit substrat et de dégradation, à partir de la partie N-terminale, dudit polypeptide ayant un résidu aromatique dans sa partie N-terminale avec

- au moins une protéine TET ayant une activité exopeptidase spécifique des résidus d'acides aminés aromatiques à l'extrémité N-terminale, ladite TET consistant en la séquence d'acides aminés telle qu'indiquée dans SEQ ID NO : 1,
- ou toute protéine homologue dérivée de ladite protéine TET telle que définie dans SEQ ID NO : 1 par substitution, ajout ou suppression d'au moins un acide aminé, à condition que la protéine dérivée conserve au moins 70 % d'identité avec la séquence d'acides aminés telle que définie dans SEQ ID NO : 1, et que ladite protéine dérivée conserve une activité d'exopeptidase spécifique des résidus d'acides aminés aromatiques à l'extrémité N-terminale,

et éventuellement une étape de récupération du contenu polypeptidique modifié d'un substrat.

8. Procédé selon la revendication 6 ou 7, dans lequel ladite étape de mise en contact est réalisée à un pH variant de 6,5 à 10.

9. Méthode selon l'une quelconque des revendications 6 à 8, dans laquelle ladite au moins une protéine TET ou ladite protéine dérivée est immobilisée sur un support solide, de préférence sur une cartouche filtrante.

10. Méthode selon l'une quelconque des revendications 6 à 8, dans laquelle ladite au moins une protéine TET ou ladite protéine dérivée est exprimée dans un hôte mésophile, à partir d'un plasmide, puis purifiée à partir de lysats cellulaires par une étape de dénaturation thermique des protéines de l'hôte.

11. Utilisation d'un support solide pour la modification de tout ou partie du contenu polypeptidique d'un substrat comprenant des peptides, des polypeptides et/ou des protéines, dans lequel ledit substrat comprend un polypeptide portant un résidu aromatique à sa partie N-terminale, dans lequel ladite modification comprend la dégradation, à partir de la partie N-terminale, dudit polypeptide portant un résidu aromatique à sa partie N-terminale et dans lequel est immobilisé sur ledit support solide :

    - au moins une protéine TET ayant une activité exopeptidase spécifique des résidus d'acides aminés aromatiques à l'extrémité N-terminale, ladite protéine TET consistant en la séquence d'acides aminés telle que définie dans SEQ ID NO : 1,
    - ou toute protéine homologue dérivée de ladite protéine TET telle que définie dans SEQ ID NO : 1 par substitution, ajout ou suppression d'au moins un acide aminé, à condition que la protéine dérivée conserve au moins 70 % d'identité avec la séquence d'acides aminés telle que définie dans SEQ ID NO : 1, et que ladite protéine dérivée conserve une activité d'exopeptidase spécifique des résidus d'acides aminés aromatiques à l'extrémité N-terminale.

12. Utilisation selon la revendication 11, dans laquelle ledit support solide est une cartouche filtrante, de la silice ou des billes magnétiques.

13. Utilisation selon la revendication 11 ou 12, dans laquelle les peptides, polypeptides et/ou protéines dudit substrat sont obtenus à partir des industries alimentaires, chimiques et de la santé, ou à partir de biomasse.

## FIG.1

## FIG.2

## FIG.3

## FIG.4

## FIG.5A

## FIG.5B

FIG.6

FIG.7

FIG.8

FIG.9

His116.B

His116.A

FIG.10

FIG.11

EP 3 724 329 B1

```
                        90           100                          110            120
MjTET    .KIGG.....IYDPTI.LNQKVVVHG.SKCDL.........IGVLGSKPPHRMK.EEEK
PhTET1   .KVGG.....IDDRLL.YGRHVNVVT.EKCIL.........DGVIGATPPHLSL..ERD
PhTET2   .PIGG.....VDPKTL.IAQRFKVWI.DKGKFI........YGVGASVPPHIQK.PEDR
PhTET3   .PIGG.....VLPETL.VAQRIRFFT.EKGER.........YGVVGVLPPHLRRGQEDK
PfTET3   .PIGG.....VLPETL.IAQKIRFFT.EKGER.........YGVVGVLPPHLRREAKDQ
DkamTET  .PIGG.....VLERTL.LYQRVVVRT.RDGRLY........RGVIGLKPPHVIK.PEEA
SpPepA   .EIGG.....WNPMVV.SSQRFKLLT.RDGHEI.......PVISGSVPPHLTRG.KGG
PaAP     EVYGGALFAPWFDRDLSLAGRVTFR..ANCKLESRLVDFRKAIAVIPNLAIHLNRAANEG
hDNPEP   ETYGGIWSTWFDRDLTLAGRVIVKCPTSGRLEQQLVHVERPILRIPHLAIHLQRNINEN
bDNPEP   ETYGGIWSTWFDRDLTLAGRVIVKCPTSGRLEQRLVHVDRPILRIPHLAIHLQRNVNEN
                                                          ▲▲▲▲▲▲▲▲ ▲▲▲▲


                        130          140                        150
MjTET    TKIIKYEDMFIDIGAESREEAI...................EMGVNIGTWVSF   SEQ ID NO:7
PhTET1   KSVIPWYDLVIDIGAESKEEAL...................ELVK.PLDFAVF   SEQ ID NO:8
PhTET2   KKAPDWDQIFIDIGAESKEEAE...................DMGVKIGTVITW   SEQ ID NO:9
PhTET3   GSKIDWDQIVVDVGASSKEEAE...................EMGFRVGTVGEF   SEQ ID NO:10
PfTET3   GGKIDWDSIIVDVGASSREEAE...................EMGFRIGTIGEF   SEQ ID NO:11
DkamTET  QKVPELRELFIDVGASSKEEVE...................KMGIRVGDIAVF   SEQ ID NO:12
SpPepA   PTMPAIADIVFDGGFADKAEAE...................SFGIRPGDTIVP   SEQ ID NO:13
PaAP     WPINAQNELPPIIAQLAPGEAA..........DFRLLLDEQLLREHGITADVLDY   SEQ ID NO:14
hDNPEP   FGPNTEMHLVPILATAIQEELEKGTPEPGPLNAVDERHHSVLMSLLCAHLGLSPKDIVEM   SEQ ID NO:15
bDNPEP   FGPNMEMHLVPILATSIQEELEKGTPEPGPLNATDERHHSVLTSLLCAHLGLSPEDILEM   SEQ ID NO:16
▲▲▲▲▲▲
```

## FIG.12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **APPOLAIRE et al.** *Biochimie*, 2015, vol. 122, 188-196
- **BILAL M** ; **IQBAL HM** ; **GUO S** ; **HU H** ; **WANG W** ; **ZHANG X**. State-of-the-art protein engineering approaches using biological macromolecules: A review from immobilization to implementation view point.. *Int J Biol Macromol.*, 02 November 2017
- **KABSCH**. Acta crystallographica. Section D. *Biological crystallography*, 2010, vol. 66, 125-132

- **GRANT, T** ; **GRIGORIEFF, N.** *Elife*, 2015, vol. 4, e06980
- **LUDTKE, S. J. et al.** *J Struct Biol*, 1999, vol. 128, 82-97
- **LUDTKE, S. J et al.** *J Struct Biol*, 1999, vol. 128, 82-97
- **ESTROZI, L. F.** ; **NAVAZA, J.** *J Struct Biol*, 2008, vol. 162, 324-334
- **EFFANTIN, G. et al.** *PLoS pathogens*, 2016, vol. 12, e1005721